(19)

(11) **EP 4 082 499 B1**

(12) **EUROPEAN PATENT SPECIFICATION**

(45) Date of publication and mention of the grant of the patent: **19.11.2025 Bulletin 2025/47**

(21) Application number: **19957128.2**

(22) Date of filing: **23.12.2019**

(51) International Patent Classification (IPC): ***A61F 13/534*** *(2006.01)*

(52) Cooperative Patent Classification (CPC): **A61F 13/534;** A61F 2013/53445

(86) International application number: **PCT/JP2019/050270**

(87) International publication number: **WO 2021/130802 (01.07.2021 Gazette 2021/26)**

(54) **ABSORBENT, AND ABSORBENT ARTICLE**

ABSORBENS UND SAUGFÄHIGER ARTIKEL

AGENT ABSORBANT, ET ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application: **02.11.2022 Bulletin 2022/44**

(73) Proprietor: **Kao Corporation**
**Tokyo 103-8210 (JP)**

(72) Inventors:
- **ONDA, Aiko**
  **Haga-gun, Tochigi 321-3497 (JP)**
- **KURAMAE, Ryota**
  **Haga-gun, Tochigi 321-3497 (JP)**

(74) Representative: **Vossius & Partner Patentanwälte Rechtsanwälte mbB**
**Siebertstraße 3**
**81675 München (DE)**

(56) References cited:
**EP-A1- 2 901 992**
**JP-A- 2017 140 461**
**JP-A- 2018 019 953**
**JP-A- 2018 050 835**
**JP-A- 2019 146 657**
**US-A1- 2013 046 263**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Technical Field

The present invention relates to an absorbent member capable of absorbing body fluid such as urine.

### Background Art

Absorbent articles such as disposable diapers and sanitary napkins are generally constituted with a topsheet that is placed in a position relatively close to the wearer's skin, a backsheet that is placed in a position relatively far from the wearer's skin, and an absorbent member that is interposed between the topsheet and backsheet. The absorbent member typically consists of a fiber stack of fiber materials such as wood pulp, and absorbent polymer particles are supported on the fiber stack in many cases. Being relatively bulky and thick, the absorbent member consisting of a fiber stack of fiber materials is excellent in cushioning properties and the like. However, unfortunately, due to the bulkiness, the absorbent article is not slim and looks unattractive, and the wearer of the absorbent article feels discomfort owing to the stiffness and the like. Especially, in a case where the wearer in the absorbent article excretes body fluids such as urine and menstrual blood, the absorbent member absorbs and retains the excretion and thus swells, which makes the above problem more serious. Therefore, studies are performed on thinning of the absorbent member.

As a conventional technique for thinning the absorbent member, for example, JP 2018-50987 A describes an absorbent member that is constituted with an upper absorbent member consisting of pulp and an absorbent polymer and a lower absorbent member that is placed so as to be adjacent to a non-skin-facing surface side of the upper absorbent member and consists of two sheets and an absorbent polymer interposed therebetween. The lower absorbent member is divided into a plurality of absorbent polymer arrangement regions each consisting of the two sheets joined together and particulate absorbent polymers sealed in the space between the sheets, and a space between the absorbent polymer arrangement regions adjacent to each other forms a flow path portion caved in from the absorbent polymer arrangement regions. In the flow path portion, the absorbent polymers do not exist or exist at a basis weight lower than that in the absorbent polymer arrangement region. Therefore, in the lower absorbent member, the absorbent polymers are not evenly distributed. According to the absorbent member described in JP 2018-50987 A, the sheet-like lower absorbent member mainly composed of an absorbent polymer is adopted so that the absorbent member can secure a sufficient absorption capacity despite being thin, and because the upper absorbent member also contains an absorbent polymer, poor liquid retentivity and occurrence of backflow, which are problems that arise in a case where the absorbent member only consists of pulp, can be solved.

JP 2010-529879 A also describes an absorbent member having a two-layer structure similar to that of JP 2018-50987 A. In the absorbent member described in JP 2010-529879 A, a lower layer relatively far from the wearer's skin in the two-layer structure substantially does not contain cellulose and is mainly constituted with an absorbent polymer. Furthermore, WO 2010/076857 A1 describes an absorbent sheet composition having a structure consisting of two or more sheets of hydrophilic nonwoven fabric and an absorbent layer that contains an absorbent resin and an adhesive and is interposed between the sheets, in which the absorbent layer is divided in a thickness direction by a water-permeable substrate.

### Summary of Invention

The present invention is an absorbent member as defined in independent claim 1. Preferred aspects are defined in the dependent claims.

In addition, the present invention is an absorbent article including the absorbent member of the present invention.

Other features, effects, and embodiments of the present invention will be described below.

### Brief Description of Drawings

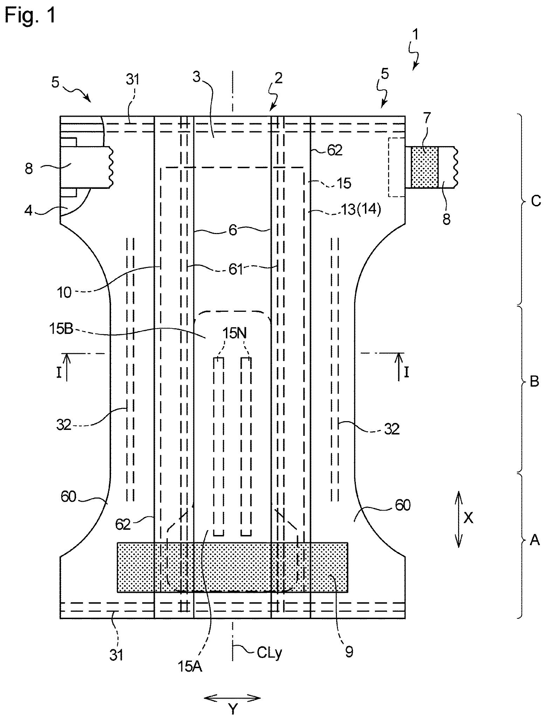

[FIG. 1] FIG. 1 is an exploded plan view schematically showing a skin-facing surface side (topsheet side) of an open type disposable diaper in its flat-out, uncontracted state, which is an embodiment of an absorbent article of the present invention. "Flat-out, uncontracted state" mentioned herein means that the diaper is made flat as shown in FIG. 1 and elastic members of each portion of the diaper in the flat-out state are stretched so that the diaper spreads out to the design dimension (the same dimension as that of the diaper planarly spread out with the complete exclusion of influences of elastic members).

[FIG. 2] FIG. 2 is a cross-sectional view schematically showing a cross section taken along the line I-I in Fig. 1.

[FIG. 3] FIG. 3 is a plan view schematically showing a skin-facing surface side of an absorbent member in the disposable diaper of FIG. 1.

[FIG. 4] FIG. 4 is a cross-sectional view schematically showing a cross section taken along the thickness direction at the central position of the absorbent member in the longitudinal direction (longitudinal centerline represented by the reference numeral CLx) shown in FIG. 3.

[FIG. 5] FIGS. 5(a) and 5(b) are cross-sectional views each schematically showing a cross section of a part of an embodiment of the absorbent member of the present invention, taken along the thickness direction.

[FIG. 6] Each of FIGS. 6(a) to 6(c) is a schematic plan view of a laminate structure on a first core side in an example or comparative examples.

[FIG. 7] FIG. 7 is a view for illustrating a method for preparing a sample for measuring a flexural rigidity variation rate BR of a laminate structure consisting of a first sheet, a first core, and an intermediate sheet (laminate structure on the first core side) per thickness variation in a longitudinal direction and a lateral direction before and after liquid absorption.

[FIG. 8] FIG. 8 is a view for illustrating a method for preparing a sample for measuring a flexural rigidity variation rate BR of a laminate structure consisting of a first sheet, a first core, and an intermediate sheet (laminate structure on the first core side) per thickness variation in an oblique direction before and after liquid absorption.

Description of Embodiments

Most of the conventional techniques relating to absorbent members are focused on how to solve the problem of deterioration of absorption performance resulting from thinning, and there has been substantially no technique focused on how to simultaneously achieve flexibility after liquid absorption and absorption performance. For example, in a case where an absorbent member that remains thin but lacks flexibility after liquid absorption is used particularly for an absorbent article such as a diaper, due to high rigidity of the absorbent member after liquid absorption, the wearer may feel discomfort. However, at present, there is substantially no technique for solving the problem. Especially, an absorbent member that is thin and has excellent properties relating to flexibility after liquid absorption has not yet been provided so far.

The present invention relates to providing an absorbent member that has excellent liquid absorption performance, is inhibited from becoming more rigid by liquid absorption, and exhibits excellent flexibility even after liquid absorption.

Hereinafter, the present invention will be described based on preferable embodiments with reference to drawings. In the description of the following drawings, the same or similar parts are marked with the same or similar reference numerals. The drawings are basically schematic views, and the ratio of dimensions may differ from the actual one in some cases.

An absorbent article of the present invention has a front-rear direction of a wearer, that is, a longitudinal direction (represented by the reference numeral "X" in the drawing) corresponding to a direction extending from the front side to the rear side through the crotch portion, and a lateral direction (represented by the reference numeral "Y" in the drawing) orthogonal to the longitudinal direction.

In the following description, unless otherwise specified, the longitudinal direction is the same direction as the longitudinal direction of an absorbent article or the longitudinal direction of a constituent member (for example, an absorbent member) of the absorbent article, and the lateral direction is the same direction as the lateral direction of an absorbent article or the lateral direction of a constituent member of the absorbent article.

The absorbent article of the present invention includes an absorbent member (represented by the reference numeral "10" in the drawing) that absorbs and retains body fluid.

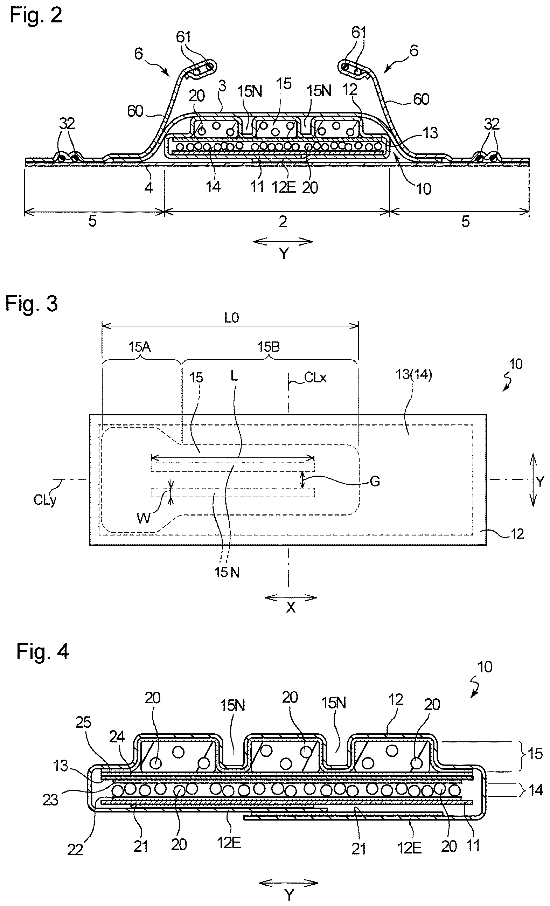

FIGS. 1 and 2 show a disposable diaper 1 which is an embodiment of the absorbent article of the present invention, and the diaper 1 has the constitution described above.

The diaper 1 is divided into three portions in a longitudinal direction X, including an crotch portion B that is placed between legs of a wearer and includes an excretory portion-facing portion (not shown in the drawing) facing an excretory portion such as penis, a front portion A that is placed closer to the front side (anterior side) of the wearer than the crotch portion B, and a rear portion C that is placed closer to the rear side (posterior side) of the wearer than the crotch portion B. Typically, the front portion A and the rear portion C each include a waistline portion that is placed around the wearer's waist when the diaper 1 is worn. The front portion A is a part of a front body portion of the diaper 1, and the rear portion C is a part of a back body portion of the diaper 1. The crotch portion B ranges from the front body portion to the back body portion in the diaper 1.

In the present invention, the front portion A, the crotch portion B, and the rear portion C can be regions determined in a case where the diaper 1 in its flat-out, uncontracted state is divided into three equal parts in a longitudinal direction X1.

The diaper 1 is a so-called open type disposable diaper. As shown in FIG. 1, the diaper 1 includes an attachment member 8 that is in the lateral side edge portions of the rear portion C of the diaper 1 in the longitudinal direction X and has an attachment portion 7, and an attachment region 9 that is on a non-skin-facing surface of the front portion A and allows the attachment portion 7 to be attached thereto.

In addition, the diaper 1 includes an absorbent assembly 2 that includes an absorbent member 10 absorbing and retaining body fluid such as urine that the wearer excretes and a flap portion 5 that extends outward from the peripheral edge of the absorbent assembly 2.

The absorbent assembly 2 includes a liquid-permeable topsheet 3 that forms a skin-facing surface, a liquid-impermeable, sparingly liquid permeable, or water repellent backsheet 4 that forms a non-skin-facing surface, and the liquid retentive absorbent member 10 that is interposed between the sheets 3 and 4. The absorbent assembly 2 has a constitution in which these members are integrated by known joining means such as adhesives. The absorbent assembly 2 extends from the front portion A to the rear portion C in the longitudinal direction X. As the topsheet 3 and the backsheet 4, those conventionally used for this type of absorbent article can be used without particular limitation. As the topsheet 3, for example, various nonwoven fabrics or porous films can be used. As the backsheet 4, for example, a resin film or a laminate of a resin film and a nonwoven fabric can be used.

In the present description, "skin-facing surface" is a surface of the absorbent article or constituent members thereof (for example, the absorbent member) that faces the wearer's skin side when the absorbent article is worn, in other words, a side that is relatively close to the wearer's skin. "Non-skin-facing surface" is a surface of the absorbent article or constituent members thereof that faces the opposite side of the skin side when the absorbent article is worn, in other words, a side that is relatively distant from the wearer's skin. "When the absorbent article is worn" mentioned herein means a state where the absorbent article is worn and stays in its normal and proper position, that is, in a right position. In FIGS. 2, 4, and 5 (cross-sectional views taken along the thickness direction) of the present application, the upper surface (the surface located on the relatively upper side) of each constituent member (for example, the absorbent member 10) is the skin-facing surface, and the lower surface (the surface located on the relatively lower side) of each constituent member is the non-skin-facing surface.

The flap portion 5 is constituted with a member extending outward from the peripheral edge of the absorbent assembly 2, and is a portion where the absorbent member is not placed. In the present embodiment, as shown in FIG. 2, the topsheet 3 covers the entire area of the skin-facing surface of the absorbent member 10, the backsheet 4 covers the entire area of the non-skin-facing surface of the absorbent member 10, and the sheets 3 and 4 extend outward in a lateral direction Y from the lateral side edges of the absorbent assembly 2 in the longitudinal direction X and form a part of the flap portion 5 (a side flap portion that extends outward in the lateral direction Y from the lateral side edges of the absorbent assembly 2 in the longitudinal direction X and virtual extended lines of the lateral side edges) together with a sheet 60 for forming a leak-proof cuff that will be described later. A plurality of members constituting the flap portion 5 is joined with each other by known joining means such as an adhesive, a heat seal, and an ultrasonic seal.

As shown in FIGS. 1 and 2, leak-proof cuffs 6 that rise up toward the wearer's skin side when the diaper is worn are placed on both sides of the absorbent assembly 2 in the longitudinal direction X. More specifically, a pair of leak-proof cuffs 6 constituted with the sheet 60 for forming a leak-proof cuff that is liquid resistant or water repellent and air permeable is placed on both sides of the skin-facing surface of the absorbent assembly 2 in the longitudinal direction X. One end side of each of the sheets 60 for forming a pair of leak-proof cuffs in the lateral direction Y is a fixed end portion that is fixed to other members (the topsheet 3 and the backsheet 4 in the embodiment shown in the drawing), and the other end side of each of the sheets 60 in the lateral direction Y is a free end portion that is not fixed to other members. In the free end portion of each of the sheets 60 for forming a leak-proof cuff, an elastic member 61 for forming a leak-proof cuff is placed which is fixed to the free end portion in a state of being stretched in the longitudinal direction X so as to be stretchable in the longitudinal direction X. When the diaper 1 is worn, due to the contractile force of the elastic member 61 for forming a leak-proof cuff, in at least the crotch portion B, the free end portion side of the sheet 60 for forming a leak-proof cuff rises up toward the wearer from a fixing part 62 fixed to other members as a base end of rising. The rising of the leak-proof cuff 6 described above prevents the excretion such as urine from leaking to the outside in the lateral direction Y.

In the flap portion 5 at the end portion of each of the front portion A and the rear portion C in the longitudinal direction X, that is, at the waist end portion, a plurality of elastic members 31 for forming waistline gathers is stretchably arranged in the lateral direction Y. The plurality of elastic members 31 is intermittently arranged at a predetermined interval in the longitudinal direction X. Because the elastic members 31 are arranged in a state of expressing elasticity as described above, a substantially continuous cyclic waist gather (waistline gather) is formed around the whole circumference of the waist end portion of each of the front portion A and the rear portion C, the waist end portion being a portion where the elastic members 31 are arranged.

Furthermore, in a leg portion of the flap portion 5 that is to be placed around the wearer's leg when the diaper 1 is worn, an elastic member 32 for forming a leg gather that is stretchable in the longitudinal direction X extends in the longitudinal direction X over the entire length of at least the crotch portion B in the longitudinal direction X. Therefore, when the diaper 1 is worn, due to the contraction of the elastic member 32, a leg gather is formed in the leg portion.

All of these elastic members 31 and 32 for forming gathers are interposed and fixed between a plurality of sheets (two sheets among the topsheet 3, the backsheet 4, and the sheet 60 for forming a leak-proof cuff in the present embodiment) constituting the flap portion 5 by joining means such as adhesives, in a state where the elastic members 31 and 32 are stretched.

The form of each of the elastic members 61, 31, and 32 described above is not particularly limited. For example, it is possible to use filamentous or stringy elastic members (for example, flat rubber bands) having a rectangular, square, circular, or polygonal cross section or to use multifilament-type filamentous elastic members.

The absorbent member 10 included in the diaper 1 is an embodiment of the absorbent member of the present invention. FIG. 3 shows the skin-facing surface side (the surface side facing the topsheet 3) of the absorbent member 10, and FIG. 4 shows a cross section of the absorbent member 10 taken along the thickness direction at the central position in the longitudinal direction X. From the viewpoint of easy understanding, in FIG. 4, the members that are actually in close contact with each other are described as being separated from each other. What is shown in FIG. 4 is not necessarily the actual condition. As shown in FIGS. 3 and 4, the absorbent member 10 includes a first sheet 11 and a second sheet 12 that face each other and an intermediate sheet 13 that is interposed between the two sheets 11 and 12. An absorbent first core 14 is interposed between the first sheet 11 and the intermediate sheet 13, and an absorbent second core 15 is interposed between the intermediate sheet 13 and the second sheet 12.

In the present embodiment, as shown in FIG. 2, the second sheet 12 side is used as a liquid-receiving surface side that firstly comes into contact with a liquid to be absorbed. That is, the absorbent member 10 is placed in the diaper 1 so that the second sheet 12 is located closer to the skin of the wearer of the diaper 1 than the first sheet 11. In the present invention, conversely, the first sheet 11 side can be used as a liquid-receiving surface side. However, considering the difference in composition between the first core 14 and the second core 15, particularly, considering the difference in an absorbent polymer occupancy and the like that will be described later, in view of improving the ability to attracting a liquid, improving liquid absorption performance, and the like, it is more preferable that the second sheet 12 side be used as a liquid-receiving surface side.

As each of the three types of sheets (the first sheet 11, the second sheet 12, and the intermediate sheet 13) included in the absorbent member 10, it is possible to use a sheet having liquid permeability or liquid absorptivity, which is typically a fiber sheet mainly composed of fiber, that is, having a fiber content more than 50% by mass. Examples of the fiber constituting the three types of sheets include natural fiber such as wood pulp including coniferous wood pulp and broad-leaved wood pulp and non-wood pulp including cotton pulp, hemp pulp, and the like; modified pulp such as cationized pulp and mercerized pulp (the above are cellulose-based fibers); synthetic fiber constituted with a resin such as polyethylene or polypropylene, and the like. One kind of each of these can be used alone, or two or more kinds of these can be used by being mixed together. Examples of forms of the three types of sheets include paper, woven fabric, and nonwoven fabric. Examples of the nonwoven fabric include air-through nonwoven fabric, heating roller nonwoven fabric, spunlace nonwoven fabric, spunbond nonwoven fabric, meltblown nonwoven fabric, and spunbond-meltblown-spunbond (SMS) nonwoven fabric. The three types of sheets are typically a single-layer structure consisting of one kind of the above material, but may be a laminate structure consisting of two or more kinds of the above materials that are laminated and integrated. The compositions and forms of the three types of sheets may be the same or different from each other.

In the present embodiment, the second sheet 12 consists of one wide sheet having a length (width) that is greater than the width of the widest portion (maximum width) of other constituent members (the first sheet 11, the intermediate sheet 13, the first core 14, and the second core 15) of the absorbent member 10 in the lateral direction Y. More specifically, the second sheet 12 of the present embodiment has a width that is not less than 2 times and not more than 3 times the aforementioned maximum width. As shown in FIG. 4, the second sheet 12 covers the entire area of the skin-facing surface of the second core 15 and extends outward in the lateral direction Y from each of the lateral side edges of the second core 15 in the longitudinal direction X, and an extension portion 12E formed as a result is wound toward the non-skin-facing surface side of the first sheet 11 and covers the entire area of the non-skin-facing surface of the first sheet. That is, the second sheet 12 covers all of the skin-facing surface and the non-skin-facing surface of the laminate structure including the first sheet 11, the intermediate sheet 13, the first core 14, and the second core 15.

In the present invention, the second sheet 12 may not have the extension portion 12E, and the width of the second sheet 12 may be approximately the same as the maximum width of the second core 15 coming into contact with the second sheet 12. Typically, except for the second sheet 12, other constituent members of the absorbent member 10 (the first sheet 11, the intermediate sheet 13, the first core 14, and the second core 15) have approximately the same maximum width, that is, approximately the same maximum length in the lateral direction Y.

The first core 14, that is, the layer interposed between the first sheet 11 and the intermediate sheet 13 contains at least an absorbent polymer 20 as an absorbent material. The first core 14 is characterized in that a proportion of the absorbent polymer in the forming material of the first core 14 is 80% by mass or more. That is, "absorbent polymer occupancy" in the first core 14 (a ratio of the mass of the absorbent polymer contained in the first core 14 to the total mass of the forming material of the core) is 80% by mass or more, and the first core 14 is mainly constituted with the absorbent polymer 20. "Forming material of the first core 14" (core forming material) mentioned herein means the substance between two sheets (specifically, the first sheet 11 and the intermediate sheet 13) located on both sides of the first core 14 that is interposed between the two sheets. Here, the core forming material does not include adhesives that are applied to the surfaces (inner surfaces) of the two sheets that face each other. Therefore, the adhesives represented by reference numerals 22 and 23 in FIG. 4 are not the core forming material of the first core 14.

The first core 14 typically contains only the absorbent polymer 20 as an absorbent material and does not contain an absorbent fiber such as wood pulp. Alternatively, even though the first core 14 contains an absorbent fiber, the content of the absorbent fiber is much smaller than (for example, not more than 20% by mass of) the content of the absorbent fiber in the second core 15.

As the absorbent polymer 20, generally, a hydrogel material capable of absorbing and retaining water can be used. For example, it is possible to use a polymer or copolymer of acrylic acid or an acrylic acid alkali metal salt. Examples thereof include polyacrylic acid and salts thereof, and polymethacrylic acid and salts thereof. Specific examples thereof include an acrylic acid polymer partial sodium salt. The shape of the absorbent polymer 20 is not particularly limited, and may be, for example, spherical, tufted, lumpy, bale-shaped, fibrous, or amorphous particles, or particles in a combined shape of these. From the viewpoint of more uniformly spraying the absorbent polymer 20 in manufacturing the first core 14 (absorbent member 10) so as to improve the liquid absorption performance of the absorbent member 10, it is preferable to use the absorbent polymer 20 as particles having the same shape. It is also preferable that the absorbent polymer 20 be spherical particles.

The adhesives 22 and 23 function to fix the absorbent polymer 20 contained in the first core 14 to the first sheet 11 and/or the intermediate sheet 13 and to join the sheets 11 and 13 together. The adhesive 22 is applied to a surface (inner surface) of the first sheet 11 that faces the intermediate sheet 13, and the adhesive 23 is applied to a surface (inner surface) of the intermediate sheet 13 that faces the first sheet 11. As the adhesives 22 and 23, the adhesives that can be used for joining members in this type of absorbent article can be used without particular limitation. Examples thereof include a hot melt adhesive. The adhesive 22 and the adhesive 23 may be the same type of adhesives or different types of adhesives. Typically, these are the same type of adhesives. The adhesives 22 and 23 will be described later.

The adhesive application pattern may vary between or be the same for the adhesive 22 on the first sheet 11 side and the adhesive 23 on the intermediate sheet 13 side. In a case where the adhesive application pattern varies between the adhesives 22 and 23, for example, an aspect may be adopted in which the adhesive on the liquid-receiving surface side firstly coming into contact with a liquid to be absorbed into the first core 14 is applied so that a non-adhesive-coated portion exists in a predetermined coated region (for example, a region where the absorbent polymer 20 exists) within the surface coated with the adhesive, and the adhesive on the opposite side of the liquid-receiving surface side is applied (by so-called solid coating) so that the non-adhesive-coated portion substantially does not exist in a predetermined coated region within the surface coated with the adhesive. Depending on the type of adhesive, application pattern, and the like, sometimes there is a concern that the liquid permeability or liquid absorption performance of the first core 14 may deteriorate. However, in a case where the adhesives are applied so that a non-adhesive-coated portion exists on the liquid-receiving surface side but substantially does not exist on the opposite side of the liquid-receiving surface side as in the above aspect, such a concern can be removed.

In the present embodiment, as shown in FIG. 2, the second sheet 12 side is used as a liquid-receiving surface side of the absorbent member 10. Therefore, in the first core 14, the intermediate sheet 13 side is a liquid-receiving surface side (the side relatively close to the skin of the wearer of the diaper 1), and the first sheet 11 side is the opposite side (the side relatively far from the skin of the wearer of the diaper 1) of the liquid-receiving surface side. Accordingly, for the adhesive 23 on the intermediate sheet 13 side, an application pattern may be adopted so that both the coated portion and non-coated portion of the adhesive 23 exist, and for the adhesive 22 on the first sheet 11 side, so-called solid coating may be adopted as an application pattern. Examples of the pattern for allowing both the coated portion and non-coated portion of the adhesive to exist include aspects in which the coated portion of the adhesive is in the form of a spiral, summit, omega, curtain, or stripe when seen in a plan view.

In the present embodiment, the adhesive is applied to two sides facing each other in the thickness direction across the first core 14, that is, to both the first sheet 11 side and the intermediate sheet 13 side. However, in the present invention, the adhesive may be applied to only one of the two sides. From the viewpoint of balance between the fixing of the absorbent polymer 20 and the liquid permeability or liquid absorption performance, the basis weight of the adhesive applied to the aforementioned two sides facing each other across the first core 14 (the total basis weight in a case where the adhesive is applied to both the first sheet 11 side and the intermediate sheet 13 side) is preferably 3 g/m$^2$ or more, and more preferably 5 g/m$^2$ or more. The basis weight is preferably 50 g/m$^2$ or less, and more preferably 30 g/m$^2$ or less.

The basis weight of the adhesive mentioned herein is the amount of the adhesive applied per unit area of the target coating region of the adhesive (specifically, for example, the skin-facing surface of the first sheet 11 or the non-skin-facing surface of the intermediate sheet 13). Therefore, for example, in a case where both the coated portion and non-coated portion of the adhesive exist in the target coating region due to the intermittent application of the adhesive, the basis weight of the adhesive in the target coating region is a value to which the non-coated portion is added.

The second core 15, that is, the layer interposed between the intermediate sheet 13 and the second sheet 12 contains at least an absorbent fiber (not shown in the drawing) and the absorbent polymer 20 as an absorbent material. Typically, the second core 15 is mainly constituted with an absorbent material. The content of the absorbent material in the second core 15 is at least 50% by mass or more, and may be 100% by mass which means that all the core forming material is an absorbent material.

As the absorbent polymer 20 in the second core 15, the same absorbent polymer as that contained in the first core 14 can be used.

Examples of the absorbent fiber include natural fiber such as wood pulp including coniferous wood pulp and broad-leaved wood pulp and non-wood pulp including cotton pulp, hemp pulp, and the like; modified pulp such as cationized pulp and mercerized pulp (the above are cellulose-based fibers); hydrophilic synthetic fiber, and the like. One kind of each of these can be used alone, or two or more kinds of these can be used by being mixed together. Typically, the absorbent fiber contained in the second core 15 is cellulose-based fiber.

The content of the absorbent fiber in the second core 15 is preferably 20% by mass or more, and more preferably 30% by mass or more. The content of the absorbent fiber is preferably 90% by mass or less, and more preferably 80% by mass or less.

In the present description, "absorbent" for a fiber is a term that those skilled in the art can easily understand, for example, just as "absorbent" mentioned about pulp. Likewise, those skilled in the art can easily understand that a thermoplastic fiber is non-absorbent. Incidentally, as for the degree of absorptivity of fiber, based on the value of moisture content measured by the following method, the relative differences of absorptivity can be compared with each other, and a more preferable range can be specified. The moisture content of the absorbent fiber is preferably 6% or more, and more preferably 10% or more. In contrast, the moisture content of a non-absorbent fiber is preferably less than 6%, and more preferably less than 4%.

<Measurement method of moisture content>

The moisture content was calculated based on the moisture content test method specified in JIS P8203. That is, a fiber sample was left to stand in a test chamber at a temperature of 40°C and a relative humidity of 80% RH for 24 hours, and then the weight W (g) of the fiber sample before an absolute drying treatment was measured in the chamber. Thereafter, the fiber sample was subjected to an absolute drying treatment by being left to stand for 1 hour in an electric dryer (for example, manufactured by ISUZUSEISAKUSHO) at a temperature of 105 ± 2°C. After the absolute drying treatment, in a test chamber under normal conditions at a temperature of 20 ± 2°C and a relative humidity of 65 ± 2%, the fiber sample wrapped in Saran wrap (registered trademark) manufactured by Asahi Kasei Corporation. is left to stand in a glass desiccator (for example, manufactured by Tech Jam Co., Ltd.) containing Si silica gel (for example, manufactured by TOYOTAKAKO Co., Ltd.) until the temperature of the fiber sample reached 20 ± 2°C. Then, a constant weight W' (g) of the fiber sample is measured, and the moisture content of the fiber sample is calculated by the following formula.

$$\text{Moisture content (\%)} = \{(W - W')/W'\} \times 100$$

The second core 15 can be manufactured according to a conventional method by using a known fiber stacking device equipped with a rotary drum. Typically, the fiber stacking device includes a rotary drum that has depressions for stacking formed on the outer peripheral surface of the drum, and a duct that has an internal flow path for conveying the core forming material (the absorbent fiber and the absorbent polymer) to the depressions for stacking. In this device, while the rotary drum is being rotated about the rotation axis along the circumferential direction of the drum, the core forming material that is conveyed by the air flow caused in the flow path due to the suction from the inside of the rotary drum is stacked in the depressions for stacking. The fiber stack formed in the depressions for stacking by this stacking step is the second core 15. Owing to the typical manufacturing method thereof described above, the second core 15 can be called "fiber stack-type absorbent core".

In the present embodiment, the second core 15 has a low-rigidity portion 15N where the core forming material (such as the absorbent fiber and the absorbent polymer) of the second core 15 does not exist or the core forming material of the second core 15 having a basis weight smaller than that of the peripheral portion exists. The term "low-rigidity portion" is derived from the fact that the core forming material-non-existing portion or the portion where the core forming material having a low basis weight exists is less rigid compared to other portions. In the present embodiment, the low-rigidity portion 15N is a through hole where no core forming material exists as shown in FIGS. 2 and 4. In a case where the low-rigidity portion 15N is not a through hole and has a basis weight lower than that of the peripheral portion, the basis weight of the low-rigidity portion 15N having a low basis weight is preferably not more than 50% and more preferably not more than 30% of the basis weight of the peripheral portion. Furthermore, on the premise that the basis weight of the low-rigidity portion 15N which is not a through hole and has a low basis weight is smaller than that of the portions other than the low-rigidity portion 15N in the second core 15, the smaller the basis weight of the low-rigidity portion 15N, the more preferable, and the basis weight of the low-rigidity portion 15N is preferably 80 g/m$^2$ or less, and more preferably 50 g/m$^2$ or less. In the most preferable aspect of the second core 15, the low-rigidity portion 15N is a through hole having zero basis weight as shown in the drawing.

In the present embodiment, as shown in FIGS. 1 and 3, the low-rigidity portion 15N is formed as a pair of portions symmetric about a lateral centerline CLy that bisects the second core 15 in the lateral direction Y and extends in the longitudinal direction X. The pair of low-rigidity portions 15N is formed on both sides of the lateral centerline CLy. Each of the pair of low-rigidity portions 15N has a shape long in the longitudinal direction X (the lengthwise direction of the second core 15), specifically, a rectangular shape when seen in a plan view.

The low-rigidity portion 15N contributes to reducing discomfort that the wearer in the diaper 1 may feel, improving liquid absorptivity or diffusivity, and the like. That is, the low-rigidity portion 15N acts as a deformation inducing portion (flexible shaft) when the second core 15 including the low-rigidity portion 15N undergoes deformation such as bending by receiving external force such as body pressure, so as to make it easier for the absorbent member 10 to be deformed in accordance with the body shape of the wearer. As a result, the discomfort that the wearer in the diaper 1 may feel can be reduced, and the wearing sensation and fit can be improved. In addition, the low-rigidity portion 15N functions as a flow path of excretion, such as urine, to be absorbed into the absorbent member 10, facilitates the diffusion of excretion in the plane direction, and can contribute to effective utilization of the liquid absorption performance of the absorbent member 10. Because the low-rigidity portion 15N plays such a role, it is preferable that the low-rigidity portion 15N be placed at a site of the second core 15 that is likely to receive external force such as body pressure and to gather the excretion. From such a viewpoint, it is preferable that the low-rigidity portion 15N be placed in a portion of the second core 15 that is located in the crotch portion B. From the same viewpoint, as shown in FIG. 3, it is preferable that the low-rigidity portion 15N extend while passing through a longitudinal centerline CLx of the absorbent member 10 in the longitudinal direction X.

The ratio of a length L (see FIG. 3) of the low-rigidity portion 15N in the longitudinal direction X (lengthwise direction) to a length L0 (see FIG. 3) of the second core 15 in the longitudinal direction X (lengthwise direction) is preferably 20% or more, and more preferably 30% or more. The ratio is preferably 95% or less, and more preferably 85% or less.

The length of the low-rigidity portion 15N in the lateral direction Y, that is, a width W (see FIG. 3) is preferably 1 mm or more, and more preferably 2 mm or more. The width W is preferably 25 mm or less, and more preferably 20 mm or less.

A gap G (see FIG. 3) between two low-rigidity portions 15N adjacent to each other in the lateral direction Y is preferably 10 mm or more, and more preferably 15 mm or more. The gap G is preferably 80 mm or less, and more preferably 60 mm or less.

The low-rigidity portion 15N is a site formed by intentionally hindering stacking of a forming material (such as an absorbent fiber and an absorbent polymer) in a step of stacking fibers of the forming material in the process of manufacturing the second core 15. The second core 15 having the low-rigidity portion 15N can be manufactured according to a conventionally known absorbent core manufacturing method. Typically, it is possible to manufacture the second core 15 by a method of obtaining the second core 15 by means of causing a forming material supplied by an air flow to be sucked into and stacked in depressions for stacking formed on the outer peripheral surface of a rotary drum, in which a part of the bottom portion of the used depressions for stacking corresponding to the low-rigidity portion 15N further protrudes outward in the radial direction of the rotary drum compared to the peripheral portion. In the second core 15 obtained by such a method, the portion where no forming material exists or the forming material having a basis weight lower than that of the peripheral portion exists is the low-rigidity portion 15N.

In the present embodiment, as shown in FIGS. 2 and 4, in a region that overlaps the low-rigidity portion 15N as a through hole when seen in a plan view, the second sheet 12 and the intermediate sheet 13 are joined together, and the intermediate sheet 13 and the first sheet 11 are joined together. In the present invention, means for joining the intermediate sheet 13 and the second sheet 12 in the low-rigidity portion 15N is not particularly limited, and may be fusion by heat sealing, ultrasonic sealing, or the like. In the present embodiment, the joining means is adhesives 24 and 25. With this constitution, in a case where the second sheet 12 side is used as a liquid-receiving surface side, a liquid (excretion) can be smoothly transferred to the first core 14 via the low-rigidity portion 15N, which makes it easier to achieve both the various performances such as liquid absorption performance and thinning. Furthermore, because a portion of the second core 15 other than the low-rigidity portion 15N comes into closer contact with the second sheet 12 and the intermediate sheet 13, the shape retentivity of the second core 15 is improved, and the shape of the second core 15 is unlikely to be ruined before and after the absorption of excretion. Accordingly, various performances of the absorbent member 10 are stably demonstrated.

The second sheet 12 and the intermediate sheet 13 do not need to be joined together in the entire region that overlaps the low-rigidity portion 15N when seen in a plan view. In other words, the entire region described above does not need to be a joined region of the sheets 12 and 13, and the sheets 12 and 13 may be joined together in at least a portion of such a region. That is, the region that overlaps the low-rigidity portion 15N when seen in a plan view can include both the portion where the second sheet 12 and the intermediate sheet 13 come into close contact with each other via the adhesives 24 and 25 and the portion where the second sheet 12 and the intermediate sheet 13 do not come into close contact with each other but are close to each other. From the viewpoint of causing the absorbent polymer to be supported in an appropriate position and securing a space allowing the absorbent polymer to swell, in the region that overlaps the low-rigidity portion 15N when seen in a plan view, the proportion of an area where the second sheet 12 and the intermediate sheet 13 are joined together and come into close contact with each other is preferably 10% or more, and more preferably 30% or more. The proportion is preferably 90% or less, and more preferably 80% or less. The above description regarding the joining of the second sheet 12 and the intermediate sheet 13 in the region that overlaps the low-rigidity portion 15N when seen in a plan view is also applicable to the joining of the intermediate sheet 13 and the first sheet 11 in such a region.

As described above, the absorbent member 10 has a laminate structure in which two absorbent layers (the first core 14 and the second core 15) are laminated together with the intermediate sheet 13 interposed therebetween, and the two layers contain the absorbent polymer 20. One of the main characteristics of the absorbent member 10 is, for example, that the absorbent member 10 satisfies the following (i) to (iii) regarding the distribution of the absorbent polymer in the laminate structure.

(i) The absorbent polymer occupancy (the ratio of the mass of the absorbent polymer contained in the first core 14 to the total mass of the core forming material) of the first core 14 is 80% by mass or more.

(ii) The absorbent polymer occupancy is higher in the first core 14 than in the second core 15. That is, regarding the absorbent polymer occupancy, a quantitative relationship of "first core 14 > second core 15" is established.

(iii) The basis weight (mass per unit area) of the absorbent polymer in the first core 14 is smaller than the basis weight of the absorbent polymer in the second core 15. That is, regarding the basis weight of the absorbent polymer, a quantitative relationship of "first **core 14** < second core 15" is established. In a case where the first or second core has a portion, such as the low-rigidity portion 15N, where no core forming material exists, "basis weight of the absorbent polymer" mentioned herein means the basis weight of the absorbent polymer in a portion other than such a portion.

As for (i) described above, the absorbent polymer occupancy in the first core 14 is at least 80% by mass or more, preferably 85% by mass or more, and more preferably 90% by mass or more. In addition, the absorbent polymer occupancy in the first core 14 may be 100% by mass.

As for (ii) described above, on the premise that absorbent polymer occupancy in first core 14 > absorbent polymer occupancy in second core 15, the ratio of absorbent polymer occupancy in first core 14/absorbent polymer occupancy in second core 15 is preferably 1.1 or more, and more preferably 1.3 or more. The ratio is preferably 5.0 or less, and more preferably 3.5 or less.

The absorbent polymer occupancy in the second core 15 is preferably 30% by mass or more, and more preferably 35% by mass or more. The absorbent polymer occupancy is preferably 90% by mass or less, and more preferably 80% by mass or less.

As for (iii) described above, on the premise that basis weight of absorbent polymer in first core 14 < basis weight of absorbent polymer in second core 15, the ratio of basis weight of absorbent polymer in second core 15/basis weight of absorbent polymer in first core 14 is preferably 1.1 or more, and more preferably 1.3 or more. The ratio is preferably 10.0 or less, and more preferably 5.0 or less.

The basis weight of the absorbent polymer in the first core 14 is preferably 60 g/m$^2$ or more, and more preferably 80 g/m$^2$ or more. The basis weight is preferably 700 g/m$^2$ or less, and more preferably 500 g/m$^2$ or less.

The basis weight of the absorbent polymer in the second core 15 is preferably 65 g/m$^2$ or more, and more preferably 70 g/m$^2$ or more. The basis weight is preferably 800 g/m$^2$ or less, and more preferably 600 g/m$^2$ or less.

In addition to satisfying the aforementioned (i) to (iii) regarding the distribution of the absorbent polymer, the absorbent member 10 is characterized in that it also satisfies (iv) in a case where BR represents a flexural rigidity variation rate of "laminate structure consisting of the first sheet 11, the first core 14, and the intermediate sheet 13" (hereinafter, also called "laminate structure on the first core side") per thickness variation before and after liquid absorption, the flexural rigidity variation rate being calculated by the following Formula (1), BR in each of two directions randomly selected from three directions consisting of two directions that are orthogonal to each other and another direction that intersects with the two direction without being orthogonal to the two directions is 5.0 or less (BR ≤ 5.0 is satisfied).

$$BR = (B_w/B_d)/T0_c \qquad (1)$$

$B_w$: Flexural rigidity of laminate structure on first core side after liquid absorption
$B_d$: Flexural rigidity of laminate structure on first core side before liquid absorption
$T0_c$: Thickness variation of laminate structure on first core side before and after liquid absorption calculated by the following Formula (2)

$$T0_c = T0_w - T0_d \qquad (2)$$

$T0_w$: Thickness of laminate structure on first core side under a load of 4.9 mN/cm$^2$ after liquid absorption
$T0_d$: Thickness of laminate structure on first core side under a load of 4.9 mN/cm$^2$ before liquid absorption

As for (iv) described above, the aforementioned "three directions" can be randomly set without particular limitation. For example, the three directions can be the longitudinal direction X (the front-rear direction of a wearer of the absorbent article in which the absorbent member is used), the lateral direction Y (a direction orthogonal to the front-rear direction of the wearer), and "a direction that intersects with the two directions X and Y at an angle of 45 degrees" (hereinafter, also called "oblique direction D"). In this case, in the absorbent member 10, at least two out of BR of the laminate structure on the first core side in the longitudinal direction X, BR of the same structure in the lateral direction Y, and BR of the same structure in the oblique direction D are 5.0 or less.

The fact that BR $\leq$ 5.0 is established in two or more directions in the laminate structure on the first core side means that the first core 14 as a main constituent of the laminate structure is inhibited from becoming more rigid by liquid absorption, that is, has a relatively small value of ($B_w/B_d$) and maintains flexibility even after liquid absorption.

As shown in the above Formula (1), BR is calculated by dividing the flexural rigidity variation rate of the laminate structure on the first core side before and after liquid absorption by the thickness variation of the laminate structure on the first core side before and after liquid absorption. Even though the thickness variation is relatively large, that is, even though the first core 14 swells to a relatively high extent after liquid absorption, in a case where the flexural rigidity variation rate is relatively low, BR $\leq$ 5.0 can be established in two or more directions. This means that in the laminate structure on the first core side in which BR $\leq$ 5.0 can be established in two or more directions, no matter whether the first core 14 swells much or little by liquid absorption, the absorbent polymer 20 is unlikely to clog the interior of the first core 14 by liquid absorption, and the first core 14 is relatively soft after swelling.

Therefore, the absorbent member 10 satisfying (iv) described above is inhibited from becoming more rigid by liquid absorption and exhibits excellent flexibility even after liquid absorption. Consequently, the diaper 1 including such an absorbent member 10 is unlikely to make the wearer feel discomfort resulting from stiffness, and makes the wearer have excellent wearing sensation not only before liquid absorption but also after liquid absorption.

The absorbent member 10 satisfies not only (iv) described above but also (i) to (iii) described above, so that the absorbent polymer occupancy and the basis weight of the first core 14 are set in the appropriate ranges described above. Therefore, the absorbent member 10 is inhibited from undergoing rigidity change by liquid absorption, and has excellent liquid absorption performance. In order to effectively inhibit the rigidity change caused by liquid absorption, it is important for the absorbent member 10 to satisfy not only (iv) described above but also (i) to (iii) described above, and for the absorbent polymer occupancy and the basis weight of the first core 14 to be set in appropriate ranges. In brief, in order to achieve the predetermined effects of the present invention, all of (i) to (iv) described above need to be satisfied.

In order that the operation and effect achieved by the satisfaction of (iv) described above are more reliably exhibited, BR in the laminate structure on the first core side in each of the aforementioned three directions is preferably 5.0 or less. For example, it is preferable that BR be 5.0 or less in all of the longitudinal direction X, the lateral direction Y, and the oblique direction D of the laminate structure on the first core side.

In the absorbent member 10, BR in each of two directions randomly selected from the aforementioned three directions of the laminate structure on the first core side is at least 5.0 or less as described above, preferably 4.8 or less, and more preferably 4.5 or less.

The lower limit of BR in each of two directions randomly selected from the aforementioned three directions of the laminate structure on the first core side is not particularly limited. However, from the viewpoint of retaining the shape of the absorbent member as far as possible, the lower limit of BR is preferably 0.1 or more, and more preferably 0.3 or more.

The values of physical properties, such as $B_w$, used for calculating BR are not particularly limited. However, in order that the predetermined effects of the present invention are more reliably exhibited, the values of physical properties are preferably set as below.

From the viewpoint of improving flexibility of the absorbent member 10, the smaller the flexural rigidity $B_w$ of the laminate structure on the first core side in the longitudinal direction X, the lateral direction Y, or the oblique direction D after liquid absorption, the more preferable. The flexural rigidity $B_w$ is preferably 200 $mN\cdot cm^2/cm$ or less, and more preferably 100 $mN\cdot cm^2/cm$ or less. The lower limit of $B_w$ is not particularly limited. However, from the viewpoint of securing shape retentivity of the absorbent member 10 and the like, the lower limit of $B_w$ is preferably 0.3 $mN\cdot cm^2/cm$ or more, and more preferably 0.5 $mN\cdot cm^2/cm$ or more.

From the viewpoint of improving flexibility of the absorbent member 10, the smaller the flexural rigidity $B_d$ of the laminate structure on the first core side in the longitudinal direction X, the lateral direction Y, or the oblique direction D before liquid absorption, the more preferable. The flexural rigidity $B_d$ is preferably 19.6 $mN\cdot cm^2/cm$ or less, and more preferably 15 $mN\cdot cm^2/cm$ or less. The lower limit of $B_d$ is not particularly limited. However, from the viewpoint of securing shape retentivity of the absorbent member 10 and the like, the lower limit of $B_d$ is preferably 0.1 $mN\cdot cm^2/cm$ or more, and more preferably 0.2 $mN\cdot cm^2/cm$ or more.

A thickness (maximum thickness) $T0_w$ of the laminate structure on the first core side under a load of 4.9 $mN/cm^2$ (= 0.5 $gf/cm^2$) after liquid absorption is preferably 1 mm or more, and more preferably 2 mm or more. $T0_w$ is preferably 30 mm or less, and more preferably 15 mm or less.

A thickness (maximum thickness) $T0_d$ of the laminate structure on the first core side under a load of 4.9 $mN/cm^2$ (= 0.5 $gf/cm^2$) before liquid absorption is preferably 0.1 mm or more, and more preferably 0.3 mm or more. $T0_d$ is preferably 5 mm or less, and more preferably 3 mm or less.

The measurement methods of the flexural rigidity $B_w$ and $B_d$ and the thicknesses $T0_w$ and $T0_d$ of the laminate structure on the first core side will be described later.

It is possible to satisfy (iv) described above, that is, to establish BR ≤ 5.0 in two or more directions in the laminate structure on the first core side, by making a more uniform distribution of the absorbent polymer 20 constituting the first core 14 of the laminate structure on the first core side. In other words, in a case where the absorbent polymer 20 is evenly distributed over the entire area of the predetermined absorbent polymer arrangement region in the first core 14, BR ≤ 5.0 can be established in two or more directions in the laminate structure on the first core side including the first core 14. That is, BR described above can be an index of the distribution state of the absorbent polymer in the laminate structure on the first core side (first core 14).

Specific examples of the aforementioned state where "absorbent polymer is evenly distributed", that is, the state where BR ≤ 5.0 is established in two or more directions in the laminate structure on the first core side include a state where the absorbent polymer 20 is arranged in the absorbent polymer arrangement region without visible gaps that can be seen by macroscopic observation when seen in a plan view (when seen in the thickness direction of the first core 14). "Without visible gaps that can be seen by macroscopic observation" mentioned herein means that although the absorbent polymer 20 is found to be arranged to evenly cover one surface (inner surface) of the first sheet 11 or the intermediate sheet 13 when the arrangement region of the absorbent polymer 20 is observed with unaided eye, it is acceptable that the gaps unintentionally formed between the absorbent polymers 20 are found when the arrangement region is microscopically observed. The size of the gap is about 10 to 1000 μm. Typically, the arrangement region of the absorbent polymer 20 in the first core 14 is substantially the entire area of the first core 14 when the first core 14 is seen in a plan view (when the first core 14 is seen in the thickness direction).

In the present embodiment, as described above, the second core 15 has the low-rigidity portion 15N (see FIG. 3 and the like), which brings about the effect of reducing the discomfort that the wearer in the diaper 1 may feel and improving the liquid absorptivity or diffusivity. In a case where the absorbent polymer is evenly distributed in the region that overlaps the low-rigidity portion 15N bringing about the above operation and effect when seen in a plan view, the operation and effect resulting from the low-rigidity portion 15N can be more reliably exhibited. Therefore, it is preferable that the absorbent polymer 20 be evenly distributed in the region that overlaps the low-rigidity portion 15N in the first core 14 when seen in a plan view. That is, it is preferable that BR ≤ 5.0 be established in at least two directions of the region that overlaps the low-rigidity portion 15N in the laminate structure on the first core side when seen in a plan view.

The absorbent member 10 of the present embodiment is used as an absorbent member of the diaper 1 which is an absorbent article to be placed in the crotch portion of a wearer. In the absorbent member used in such an absorbent article, it is particularly important for the region placed in the crotch portion of a wearer of the absorbent article to have various characteristics such as liquid absorption performance or flexibility after liquid absorption. Therefore, it is preferable that the absorbent polymer be evenly distributed in such a region. From such a viewpoint, it is preferable that the absorbent polymer 20 be evenly distributed in the region of the first core 14 that will be placed in the crotch portion of the wearer when the diaper 1 is worn. That is, it is preferable that BR ≤ 5.0 be established in at least two directions of the region to be located in the crotch portion B in the laminate structure on the first core side.

In order that the operation and effect brought about by the satisfaction of (iv) described above are more reliably exhibited, the ratio between the values of $B_w$ in two directions randomly selected from the aforementioned three directions is preferably 0.8 or more and 1.2 or less, and more preferably 0.9 or more and 1.1 or less. For example, in the laminate structure on the first core side, each of "$B_w$ in longitudinal direction X/ $B_w$ in lateral direction Y", "$B_w$ in longitudinal direction X/ $B_w$ in oblique direction D", and "$B_w$ in lateral direction Y/ $B_w$ in oblique direction D" is preferably in the range described above. The ratio between the values of $B_w$ in two directions randomly selected from the aforementioned three directions can be made 0.8 or more and 1.2 or less, by making a more uniform distribution of the absorbent polymer 20 constituting the first core 14 of the laminate structure on the first core side.

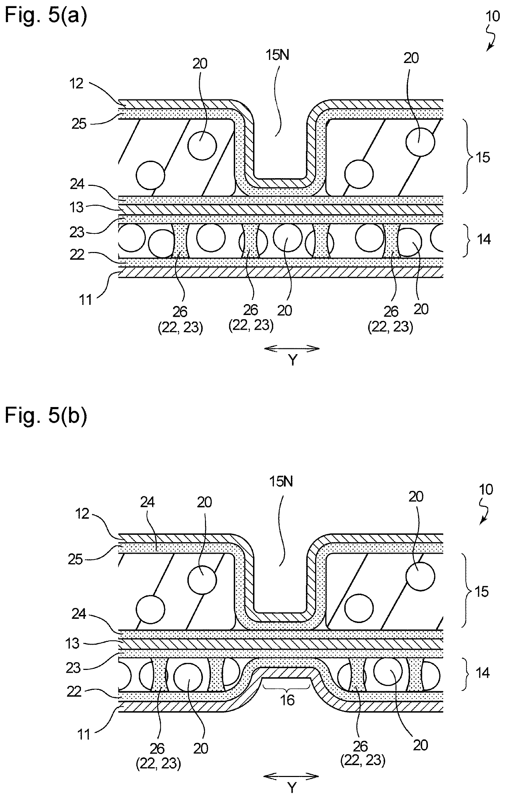

More specifically, as for the absorbent member 10, in the laminate structure on the first core side, the first sheet 11 and the intermediate sheet 13 are joined together via the adhesives 22 and 23. One of the examples of the way the sheets 11 and 13 are joined together is an aspect in which the sheets 11 and 13 are joined together via a columnar portion 26 consisting of the adhesive 22 on the first sheet 11 side and/or the adhesive 23 on the intermediate sheet 13 side as shown in FIG. 5(a). In a case where the sheets are joined together via such a columnar portion 26, the sheets 11 and 13 are in a state of being close to each other at a predetermined interval without coming into close contact with each other. Typically, a plurality of columnar portions 26 is formed in the shape of regularly or irregularly scattered dots in the coated region of the adhesives 22 and 23 (region where the absorbent polymer 20 exists) when the laminate structure on the first core side is seen in a plan view (when the laminate structure on the first core side is seen in the thickness direction). In some cases, the plurality of columnar portions 26 can have the same height (the length of the first core 14 in the thickness direction) or different heights. The plurality of columnar portions 26 can include both the columnar portion 26 that is joined with the absorbent polymer 20 interposed between the sheets 11 and 13 (columnar portion 26 joined with each of the sheets 11 and 13 and the absorbent polymer 20) and the columnar portion 26 that is not joined with the absorbent polymer 20 (columnar portion 26 joined only with the sheets 11 and 13). The formation of the columnar portion 26 can be controlled by appropriately adjusting the type of the adhesive to be used, the amount of the adhesive to be applied, the basis weight or particle size of the absorbent polymer 20 to be interposed between the sheets 11 and 13, and the like.

As shown in FIG. 5(b), sometimes the laminate structure on the first core side can include both the portion where the first sheet 11 and the intermediate sheet 13 are joined together via the columnar portion 26 and a portion 16 where the sheets 11 and 13 come into close contact with each other via the adhesives 22 and 23. In FIG. 5(b), the close contact portion 16 of the sheets 11 and 13 is formed in a region that overlaps the low-rigidity portion 15N when seen in a plan view. However, the site where the close contact portion 16 is to be formed is not particularly limited, and a plurality of close contact portions 16 in the shape of regularly or irregularly scattered dots can be formed in the coated region of the adhesives 22 and 23 (the region where the absorbent polymer 20 exists). Here, from the viewpoint of making sure that the absorbent polymer 20 is evenly distributed in the first core 14, it is preferable that the close contact portion 16 of the sheets 11 and 13 do not exist or only few close contact portions 16 exist.

As the adhesive that facilitates the formation of the columnar portion 26, it is preferable to use a flexible adhesive that can stretch in accordance with the swelling-induced change caused by the liquid absorption of the absorbent polymer 20. Examples of such raw materials include an acrylic adhesive containing one or more kinds of monomers such as (co) polymers of vinyl monomers (such as an ethylene-vinyl acetate copolymer) including 2-ethylhexyl acrylate, butyl acrylate, ethyl acrylate, cyanoacrylate, vinyl acetate, and methyl methacrylate, a silicone-based adhesive containing a polydimethylsiloxane polymer or the like, a rubber-based adhesive such as a natural rubber-based adhesive containing natural rubber or the like, an isoprene-based adhesive containing one or more kinds of polymers such as polyisoprene and chloroprene, a styrene-based adhesive containing one or more kinds of copolymers such as a styrene-butadiene copolymer (SBR), a styrene-isoprene-styrene block copolymer (SIS), a styrene-butadiene-styrene block copolymer (SBS), a styrene-ethylene-butadiene-styrene block copolymer (SEBS), and a styrene-ethylene-propylene-styrene block copolymer (SEPS), and the like. One kind of each of these may be used alone, or two or more kinds of these may be used in combination.

As the adhesives 22 and 23 to be used in the first core 14, among these adhesives, a rubber-based adhesive is preferably used, and a styrene-based adhesive is more preferably used among rubber-based adhesives, because such an adhesive has excellent flexibility and elasticity, allows the first sheet 11 and the intermediate sheet 13 to remain joined together even after the absorbent polymer 20 swells, and exerts contractile force that allows the absorbent polymer 20 to be easily retained between the sheets 11 and 13.

From the viewpoint of achieving both the flexibility of the adhesive and the adhesiveness to a sheet, the adhesives 22 and 23 used for the first core 14 are preferably hot melt adhesives. As the hot melt adhesives, for example, it is possible to use adhesives obtained by incorporating a tackifier such as a petroleum resin or a polyterpene resin and a plasticizer such as paraffin-based oil and optionally incorporating an antioxidant based on phenol, amine, phosphorus, benzimidazole, and the like into the various adhesives described above.

At 50°C, the relaxation time of the adhesives 22 and 23 used in the first core 14 that is obtained by viscoelasticity measurement is preferably 1 second or longer, more preferably 2 seconds or longer, and even more preferably 3 seconds or longer. In a case where the relaxation time of the adhesives 22 and 23 is as described above, appropriate flexibility and elasticity are exhibited, which allows the first sheet 11 and the intermediate sheet 13 to remain joined together and allows the absorbent polymer 20 to be retained between the sheets 11 and 13 even after the absorbent polymer 20 swells.

The relaxation time of the adhesive obtained by viscoelasticity measurement at 50°C is calculated as the reciprocal of the value of tan $\theta$ obtained when the dynamic viscoelasticity of the adhesive is measured under the following conditions. Specifically, a rotary rheometer (manufactured by Anton Paar GmbH, "Physica MCR301") is used, and the adhesive (adhesives 22 and 23) as a measurement target is interposed between a support plate that supports the measurement sample from below and is in the form of a circle when seen in a plan view and a presser plate that is placed above the support plate to face the support plate and is in the form of a circle when seen in a plan view. In this state, the adhesive is in the form of a circle when seen in a plan view, and has a thickness of 1.5 mm and a diameter of 12 mm. The frequency for measurement is set to 1 Hz, the strain amplitude is set to 0.05%, the cooling rate is set to 2°C/min, and the measurement is performed in a temperature ranging from 120°C to -10°C. tan $\theta$ is a value obtained by dividing a loss modulus G'' by a storage modulus G'.

In the present embodiment, as shown in FIG. 4, the first sheet 11 and the extension portion 12E of the second sheet 12 are joined together via an adhesive 21, the intermediate sheet 13 and the second core 15 are joined together via the adhesive 24, and the second sheet 12 and the intermediate sheet 13 are joined together via the adhesives 24 and 25. In a case where the constituent members of the absorbent member 10 are integrated via adhesives in this way, the appearance of the absorbent member 10 is more stabilized, and the predetermined performance can be more stably demonstrated. The adhesive 24 is applied to a surface (the skin-facing surface in the present embodiment) of the intermediate sheet 13 opposite to a surface facing the first sheet 11, and the adhesive 25 is applied to a surface (non-skin-facing surface in the present embodiment) of the second sheet 12 facing the second core 15 or the intermediate sheet 13. As the adhesives 21, 24, and 25, the adhesives that can be used for joining members in this type of absorbent article can be used without particular limitation. Examples thereof include a hot melt adhesive. The amount of the adhesives 21, 24, and 25 applied and the application pattern of the adhesives are not particularly limited, and can be appropriately set.

As shown in FIG. 3, the absorbent member 10 has a shape long in one direction when seen in a plan view. In the present embodiment, the absorbent member 10 has a shape long in the longitudinal direction X of the diaper 1, specifically, a rectangular shape. The lengthwise direction of the absorbent member 10 coincides with the longitudinal direction X, and the width direction of the absorbent member 10 orthogonal to the lengthwise direction coincides with the lateral direction Y. In addition, the first core 14 and the second core 15 (intermediate sheet 13) also have a shape long in the longitudinal direction X. In the present embodiment, the second core 15 and the intermediate sheet 13 have substantially the same shape when seen in a plan view.

In the present embodiment, as shown in FIG. 3, based on the center of the absorbent member 10 in the lengthwise direction, that is, based on the virtual longitudinal centerline CLx that bisects the absorbent member 10 in the longitudinal direction X and extends in the lateral direction Y as a boundary, one side (left side in FIG. 3) of the absorbent member 10 in the lengthwise direction (longitudinal direction X) is richer in the absorbent material (such as the absorbent polymer and the absorbent fiber) than the other side (right side in FIG. 3) of the absorbent member 10 in the lengthwise direction (longitudinal direction X). Typically, the core forming material of the absorbent member 10 substantially consists solely of the absorbent material. Specifically, for example, the ratio of the absorbent material to the total mass of the core forming material is 100% by mass. Therefore, in a case where the absorbent material is concentrated in a part of the absorbent member 10, the concentration of the absorbent material is reflected in the appearance of the absorbent member 10 as shown in FIG. 3. Accordingly, in a case where the core forming material substantially consists solely of the absorbent material, "absorbent material" in "one side of the absorbent member 10 in the lengthwise direction is richer in the absorbent material than the other side of the absorbent member 10 in the lengthwise direction" can be called "core forming material" in other words.

Examples of the aspect in which the absorbent material (core forming material) is concentrated in a certain part of the absorbent member 10 include 1) aspect in which the absorbent material is concentrated in one side of the first core 14 in the lengthwise direction (longitudinal direction X) but is evenly distributed in the second core 15, 2) aspect in which the absorbent material is concentrated in one side of the second core 15 in the lengthwise direction (longitudinal direction X) but is evenly distributed in the first core 14, 3) aspect in which the absorbent material is concentrated in one side in the lengthwise direction (longitudinal direction X) in both the cores 14 and 15, and 4) aspect in which the absorbent material is concentrated in one side in the lengthwise direction (longitudinal direction X) in one of the cores 14 and 15 and concentrated in the other side in the lengthwise direction (longitudinal direction X) in the other of the cores 14 and 15, but the absorbent material is concentrated overall in one side in the lengthwise direction (longitudinal direction X) in the absorbent member 10. The aforementioned "absorbent material (core forming material) is evenly distributed" means a state where the absorbent material (core forming material) is found to be uniformly distributed in general in a case where the first core 14, the second core 15, or the absorbent member 10 is macroscopically observed (for example, in a case where the first core 14 or the like is observed with the unaided eye), and uniformity in a strict sense that can be firstly checked in a case where a part of the first core 14 or the like is microscopically observed (for example, in a case where a cross section of the first core 14 or the like is observed with a microscope) is not a problem.

In the present embodiment, as shown in FIG. 3, the aspect 2) is adopted as the aspect in which the absorbent material (core forming material) is concentrated in a certain part. That is, in the second core 15, a portion (left side in FIG. 3) that extends while passing through the center (longitudinal centerline CLx of the absorbent member 10) in the lengthwise direction (longitudinal direction X) along the same direction and located on one side of the second core 15 in the lengthwise direction (longitudinal direction X) of the absorbent member 10 is richer in the absorbent material (core forming material), than a portion (right side in FIG. 3) that is located on the other side of the second core 15 in the lengthwise direction (longitudinal direction X) of the absorbent member 10. More specifically, in a case where the absorbent member 10 is divided into a front side (a portion closer to the front side of the wearer of the diaper 1) and a rear side (a portion closer to the rear side of the wearer of the diaper 1) based on the longitudinal centerline CLx as a boundary, in the second core 15, more absorbent material (core forming material) is in the portion located on the front side than in the portion located on the rear side. On the other hand, in the first core 14, the absorbent material (core forming material) is evenly distributed.

In the present embodiment, as shown in FIG. 3, the area of the main surface of a portion (left side in FIG. 3) located on one side of the second core 15 in the lengthwise direction (longitudinal direction X) of the absorbent member 10 is larger than the area of the main surface of a portion (right side in FIG. 3) located on the other side of the second core 15 in the lengthwise direction (longitudinal direction X) of the absorbent member 10. "Main surface" mentioned herein means a surface having the largest area in the second core 15. Usually, it is either a liquid receiving surface that firstly comes into contact with a liquid to be absorbed or a surface that is on the opposite side of the liquid receiving surface, or either a skin-facing surface or a non-skin-facing surface. Unless otherwise specified, the definition of the main surface is also applied to other members such as the first core 14 and the absorbent member 10.

More specifically, in a plan view as shown in FIG. 3, the first core 14 (intermediate sheet 13) has a rectangular shape, the area of the main surface of the first core 14 is larger than that of the second core 15, and the second core 15 is placed so as to be included in the region surrounded by the peripheral edge of the first core 14 (intermediate sheet 13) and does not extend outward from the peripheral edge of the first core 14.

In the lengthwise direction (longitudinal direction X), the length of the second core 15 is smaller than the length of the first core 14 (intermediate sheet 13). On the premise that length of second core 15 in longitudinal direction X < length of first core 14 in longitudinal direction X, the ratio of length of first core 14 in longitudinal direction X/length of second core 15 in longitudinal direction X is preferably 1.05 or more, and more preferably 1.1 or more. The ratio is preferably 1.7 or less, and more preferably 1.6 or less.

The length (width) of the second core 15 in the width direction (lateral direction Y) is not uniform over the entire length of the second core 15 in the lengthwise direction. The second core 15 has, in the lengthwise direction (longitudinal direction X), a broad portion 15A that is relatively wide and has a large-area main surface and a narrow portion 15B that is relatively narrow and has a small-area main surface.

The width (length in the lateral direction Y) of a portion of the broad portion 15A that is close to the narrow portion 15B gradually decreases toward the narrow portion 15B side.

Within the intermediate sheet 13, in the peripheral portion of the surface on which the second core 15 is placed (skin-facing surface of the intermediate sheet 13 in the present embodiment), particularly, on the outer side of the narrow portion 15B in the lateral direction Y, there is a portion where the second core 15 is not placed and the intermediate sheet 13 is exposed. In the portion where the intermediate sheet 13 is exposed, the intermediate sheet 13 and the second sheet 12 are joined together via the adhesives 24 and 25 (see FIG. 4).

The ratio of maximum length (maximum width) of broad portion 15A in lateral direction Y/maximum width of narrow portion 15B in lateral direction Y is preferably 1.1 or more, and more preferably 1.2 or more. The ratio is preferably 5.5 or less, and more preferably 3.0 or less.

The ratio of length of broad portion 15A in longitudinal direction X/length of narrow portion 15B in longitudinal direction X is preferably 1.2 or more, and more preferably 1.5 or more. The ratio is preferably 7.0 or less, and more preferably 5.0 or less.

In the present embodiment, as shown in FIG. 1, the absorbent member 10 is placed so that the side on which the absorbent material (core forming material) is concentrated in the lengthwise direction of the absorbent member 10, that is, the broad portion 15A side of the second core 15 is located close to the front portion A of the diaper 1. In the absorbent member 10 as a constituent member of the diaper 1, usually, the body fluid to be absorbed is concentrated in a specific site. Specifically, the body fluid is concentrated in the crotch portion B or the central portion of the diaper 1 in the longitudinal direction X or concentrated in a region close to the crotch portion B in the front portion A in addition to the aforementioned portions. Therefore, it is preferable that the absorbent member 10 be placed so that the portion of the absorbent member 10 relatively rich in the absorbent material (core forming material) is located in such regions in which the body fluid is concentrated. In a case where the absorbent member 10 is placed as above, it is possible to achieve both the liquid absorption performance and thinning of the absorbent member 10.

Regarding a Klemm absorption height measured according to JIS P 8141, it is preferable that a quantitative relationship of "first sheet 11 ≤ second sheet 12 < intermediate sheet 13" be established, and that the Klemm absorption height of the intermediate sheet 13 be 20 mm or more and preferably 25 mm or more for 5 minutes. In a case where the quantitative relationship is established, a liquid is rapidly absorbed and diffused in the plane direction, which is mainly effective for increasing the utilization efficiency of the absorbent polymer 20 of the first core 14. The Klemm absorption height of the intermediate sheet 13 that is in the above range is mainly effective for making a liquid diffusing better in the plane direction. Therefore, the absorbent member 10 satisfying these conditions is particularly excellent in the liquid absorption performance. The Klemm absorption height is an index of liquid retentivity. The higher the Klemm absorption height, the higher the liquid retentivity of a fiber sheet.

From the viewpoint of establishing the aforementioned quantitative relationship regarding the Klemm absorption height, crepe paper is particularly preferable as the intermediate sheet 13. Crepe paper may also be used as the first sheet 11 and the second sheet 12. Crepe paper is paper with wrinkles and creases. Because the wrinkles or creases result in elasticity, when stretched, the crepe paper has a larger surface area than general paper. Due to these characteristics of the crepe paper, the intermediate sheet 13 and the like consisting of the crepe paper have a relatively high Klemm absorption height, which results in a high ability to attract a liquid, and the intermediate sheet 13 and the like having absorbed a liquid exhibit relatively low rigidity. The degree of crepe in crepe paper is represented by a crepe ratio measured by an underwater elongation method, which is preferably 5% or more, more preferably 10% or more, and even more preferably 15% or more. In addition, a crepe ratio of 30% or less is practical.

One of the preferable examples of the intermediate sheet 13 is crepe paper having a crepe ratio in the aforementioned preferable range.

Incidentally, the crepe ratio of the second sheet 12 is preferably less than 1%. In a case where the crepe ratio of the second sheet 12 is in this range, the Klemm absorption height of the second sheet 12 can be lower than that of the intermediate sheet 13, a liquid is unlikely to be retained on the inside of the second sheet 12, and air permeability can be improved.

The crepe ratio of the first sheet 11 may be in the same range as that of the second **sheet 12.**

The crepe ratio can be measured by an underwater elongation method, for example, based on the following method. The crepe ratio is measured at a temperature of 23 ± 2°C and a relative humidity of 50 ± 5%, by using a sample that is kept in the same environment for 24 hours or more before the measurement. The sheet as a measurement target is cut in a size of 25 mm in a direction in which creases extend x 100 mm in a direction orthogonal to the above direction, thereby preparing a measurement sample. The measurement sample is immersed in water for 1 minute and then pulled up, and the crepe ratio is calculated by the following formula, from the change in size in the aforementioned orthogonal direction. The sample is measured three times, and the arithmetic mean thereof is adopted as the crepe ratio (%). **In** a case where the size of 100 mm cannot be secured in the orthogonal direction, it is possible to cut the sample in a size of at least 30 mm or more in the orthogonal direction and to calculate the crepe ratio.

Crepe ratio (%) = {(dimension after immersion in water (mm))/(dimension before immersion in water (mm)) - 1} × 100

For example, nonwoven fabric is a preferable embodiment of the first sheet 11, and SMS nonwoven fabric is more preferable embodiment thereof.

For example, as a preferable embodiment of the second sheet 12, the second sheet 12 may be constituted with nonwoven fabric. More specifically, examples thereof include spunbond nonwoven fabric and SMS nonwoven fabric. Among these, SMS nonwoven fabric is particularly preferable.

Examples of a preferable embodiment of the intermediate sheet 13 include paper mainly composed of cellulose-based fiber (paper in which the content of cellulose-based fiber is 50% by mass or more).

The basis weight of the first sheet 11 is preferably 5 g/m$^2$ or more, and more preferably 8 g/m$^2$ or more. The basis weight is preferably 80 g/m$^2$ or less, and more preferably 60 g/m$^2$ or less.

The basis weight of the second sheet 12 is preferably 5 g/m$^2$ or more, and more preferably 8 g/m$^2$ or more. The basis weight is preferably 80 g/m$^2$ or less, and more preferably 60 g/m$^2$ or less.

The basis weight of the intermediate sheet 13 is preferably 5 g/m$^2$ or more, and more preferably 8 g/m$^2$ or more. The basis weight is preferably 80 g/m$^2$ or less, and more preferably 60 g/m$^2$ or less.

The basis weight of the laminate structure on the first core side, that is, the basis weight of "laminate structure consisting of the first sheet 11, the first core 14, and the intermediate sheet 13" is not particularly limited. From the viewpoint of balance between absorption capacity, thinning, and flexibility, the basis weight is preferably 80 g/m$^2$ or more, and more preferably 100 g/m$^2$ or more. The basis weight is preferably 600 g/m$^2$ or less, and more preferably 400 g/m$^2$ or less.

From the same viewpoint, the basis weight of the second core 15 (basis weight of the portion other than the low-rigidity portion 15N that will be described later) is preferably 100 g/m$^2$ or more, and more preferably 150 g/m$^2$ or more. The basis weight is preferably 800 g/m$^2$ or less, and more preferably 700 g/m$^2$ or less.

The absorbent member 10 can be manufactured according to the conventional method using a known device. The manufacturing apparatus of the absorbent member 10 typically includes a first manufacturing unit that manufactures the first core 14, and a second manufacturing unit that manufactures the second core 15. The second manufacturing unit typically includes the fiber stacking device described above. The first manufacturing unit typically includes sheet conveying means, application means for applying an adhesive to the sheet, and spray means for the absorbent polymer 20. In the first manufacturing unit, while either the first sheet 11 or the intermediate sheet 13 is being conveyed by the conveying means, an adhesive is applied to one surface of the sheet being conveyed in a predetermined pattern by using the application means. In addition, the absorbent polymer 20 is sprayed by the spray means on the coated region of the adhesive in the sheet being conveyed, one of the sheets 11 and 13 is then superposed on one surface of the other on which the absorbent polymer 20 is sprayed, the sheets are integrated via the adhesive, and the obtained material is cut in a unit length of a product if necessary, thereby manufacturing the first core 14.

In the manufacturing apparatus of the absorbent member 10, the second core 15 manufactured in the second manufacturing unit is placed on a surface of the second sheet 12 to which the adhesives 21 and 25 are applied in advance, the surface being coated with the adhesives. In this state, while the second core 15 is being conveyed together with the second sheet 12, the first core 14 manufactured in the first manufacturing unit is superposed on the second core 15 being conveyed, the first core 14 and the second core 15 are integrated via the adhesive 24 that is applied in advance to at least one of the cores 14 and 15, the extension portion 12E of the second sheet 12 (see FIG. 4) is then wound toward the first core 14 side so that the first core 14 is covered, and the obtained material is cut in the unit length of a product if necessary, thereby manufacturing the absorbent member 10.

The use of the absorbent member of the present invention is not limited to the absorbent member of an absorbent article described above. The absorbent member of the present invention can be widely used for absorbing an aqueous liquid.

The absorbent article of the present invention is not limited to the open type disposable diaper described in the present embodiment, and includes a wide variety of articles used for absorbing body fluid (such as urine, menstrual blood, loose stool, or sweat) discharged from the human body. The absorbent article of the present invention also includes disposable pull-on diapers having no attachment structure such as the attachment member 8 and the attachment region 9, sanitary napkins, sanitary shorts, and the like.

Hitherto, the present invention has been described based on preferable embodiments thereof. However, the absorbent article of the present invention is not limited to the embodiments, and can be appropriately modified.

For example, in the above embodiments, no other members are interposed between the second core 15, the second sheet 12, and the intermediate sheet 13. However, a permeable or liquid-absorptive member may be interposed between the second core 15 and the second sheet 12 and/or between the second core 15 and the intermediate sheet 13. Examples thereof include an aspect in which either or both of the skin-facing surface and non-skin-facing surface of the second core 15 are covered with a liquid-permeable or liquid-absorptive sheet separate from the second sheet 12 and the intermediate sheet 13.

In the above embodiments, each of the first sheet 11, the second sheet 12, and the intermediate sheet 13 is a separate and independent member. However, any two of these may constitute one sheet. Specific examples thereof include an aspect in which the absorbent member 10 includes one sheet that covers the skin-facing surface (the second core 15 side) and the non-skin-facing surface (opposite side of the second core 15 side) of the first core 14, the portion of the one sheet that covers the non-skin-facing surface of the first core 14 is the first sheet 11, and the portion of the one sheet that covers the skin-facing surface of the first core 14 is the intermediate sheet 13. Another specific example thereof is an aspect in which the absorbent member 10 includes one sheet that covers the skin-facing surface (opposite side of the first core 14 side) and the non-skin-facing surface (the first core 14 side) of the second core 15, the portion of the one sheet that covers the non-skin-facing surface of the second core 15 is the intermediate sheet 13, and the portion of the one sheet that covers the skin-facing surface of the second core 15 is the second sheet 12.

The second core 15 may not have the low-rigidity portion 15N. Even though the second core 15 has the low-rigidity portion 15N, the second sheet 12 and the intermediate sheet 13 do not need to be joined together in a region that overlaps the low-rigidity portion 15N when seen in a plan view. However, in order that the predetermined effects of the present invention are exhibited, it is effective for the sheets 12 and 13 to be joined together in the low-rigidity portion 15N as in the embodiments described above. Furthermore, the shape of the low-rigidity portion 15N in a plan view and the number of the low-rigidity portions 15N are not limited to the above embodiments, and can be arbitrarily set. For example, one low-rigidity portion 15N having a rectangular shape when seen in a plan view may extend in the longitudinal direction X at the center of the second core 15 in the lateral direction Y, or a plurality of low-rigidity portions 15N, for example, three or more low-rigidity portions 15N having a rectangular shape when seen in a plan view may be intermittently arranged in the lateral direction Y so that the lengthwise direction thereof coincides with the longitudinal direction X.

Furthermore, in the absorbent article of the present invention, from the viewpoint of improving absorptivity, leakproofness, and the like, a liquid-permeable sheet called a second sheet, sublayer sheet, or the like that consists of paper or various nonwoven fabrics may be interposed between the topsheet 3 and the absorbent member 10 (second sheet 12).

All the parts included in only one embodiment described above can be mutually used as appropriate. The embodiments of the present invention described above also include the following aspects.

Hereinafter, the present invention will be more specifically described with reference to examples, but the present invention is not limited to the examples.

[Example 1 and Comparative Examples 1 and 2]

An open type disposable diaper basically having the same constitution as the diaper 1 shown in FIG. 1 was prepared according to a conventional method. The absorbent assembly of the prepared disposable diaper includes a topsheet that is closest to the wearer's skin, an absorbent member that is the second closest to the wearer's skin, and a backsheet that is farthest from the wearer's skin. As the topsheet, a hydrophilic air-through nonwoven fabric having a basis weight of 25 g/m$^2$ was used. As the backsheet, a laminate of a resin film and a nonwoven fabric was used, in which the skin-facing surface side consisted of a polyethylene resin film having a basis weight of 18 g/m$^2$ and the non-skin facing surface side consisted of a hydrophobic air-through nonwoven fabric having a basis weight of 25 g/m$^2$. As the absorbent member, an absorbent member having the same constitution as the aforementioned absorbent member 10 was used. An SMS nonwoven fabric having a basis weight of 10 g/m$^2$ was used as the first sheet. An SMS nonwoven fabric having a basis weight of 10 g/m$^2$ was used as the second sheet. Tissue having a basis weight of 16 g/m$^2$ (the same one as the mount for wrapping the absorbent core in this type of absorbent article) was used as the intermediate sheet.

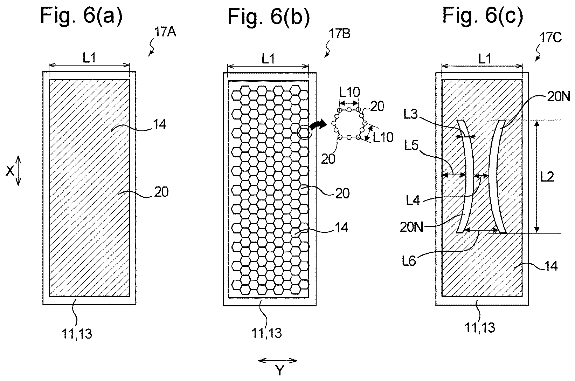

In the process of manufacturing the first core, the method of spraying the absorbent polymer (the amount of the absorbent polymer sprayed per unit time, spray pattern, or the like) was appropriately changed so that three types of first cores (see FIG. 6) in which the absorbent polymer was sprayed in different states were manufactured. The absorbent polymer occupancy was 100% in all of the three types of first cores.

As the second core, a fiber stack containing an absorbent fiber (needle bleached kraft pulp; NBKP) and an absorbent polymer was used. For the second core, low-rigidity portions in the form of through holes were made, and the second sheet and the intermediate sheet were joined together in a region that overlapped the low-rigidity portions when seen in a plan view. Only one type of second core was prepared and used for all the example and comparative examples.

By using one of the three types of first cores and one type of second core described above, a plurality of open type disposable diapers in which the absorbent polymer was distributed in the first core in different states was prepared and used as an example or comparative example.

FIG. 6 shows a schematic constitution of a laminate structure on the first core side (laminate structure consisting of the first sheet 11, the first core 14, and the intermediate sheet 13) in each of the example and comparative examples. In FIG. 6, from the viewpoint of easy understanding, a sheet (the first sheet 11 or the intermediate sheet 13) on one side covering the first core 14 (absorbent polymer 20) is not illustrated.

In a laminate structure 17A on the first core side of Example 1, as shown in FIG. 6(a), the entire region of the first sheet 11 and the intermediate sheet 13 that have the same rectangular shape when seen in a plan view and have the same dimension is provided with the absorbent polymer 20 evenly sprayed substantially without a gap, except for the peripheral portion of the first sheet 11 and the intermediate sheet 13. In this way, the first core 14 is formed between the sheets 11 and 13. In the laminate structure 17A on the first core side, the basis weight of the absorbent polymer 20 in the first core 14 was 110 g/m$^2$, and a length L1 of the first core 14 in the lateral direction Y was 110 mm.

In a laminate structure 17B on the first core side of Comparative Example 1, as shown in FIG. 6(b), the entire region of the first sheet 11 and the intermediate sheet 13 that have the same rectangular shape when seen in a plan view and have the same dimension is provided with the absorbent polymer 20 intermittently sprayed in a predetermined pattern, except for the peripheral portion of the first sheet 11 and the intermediate sheet 13. In this way, the first core 14 is formed between the sheets 11 and 13. Specifically, the spraying pattern of the absorbent polymer 20 in the laminate structure 17B on the first core side is a so-called honeycomb shape in which regular hexagons are arranged without gaps when seen in a plan view. The absorbent polymer 20 is arranged along each of the regular hexagons. In the laminate structure 17B on the first core side, the basis weight of the absorbent polymer 20 in the first core 14 was 100 g/m$^2$, and the length L1 of the first core 14 in the lateral direction Y was 110 mm. The length (length of one side of each regular hexagon) represented by the reference numeral L10 in FIG. 6(b) was 8 mm.

In a laminate structure 17C on the first core side of Comparative Example 2, as shown in FIG. 6(c), a pair of non-sprayed regions 20N of the absorbent polymer 20 extending in the longitudinal direction X is formed at the central portion of the sheets 11 and 13 in the longitudinal direction X. When seen in a plan view, each of the pair of non-sprayed regions 20N is in the form of a projection protruding toward the other non-sprayed region 20N, and the apex of the projection is located at the central portion of the non-sprayed region 20N in the lengthwise direction (longitudinal direction X). In the laminate structure 17C on the first core side, the basis weight of the absorbent polymer 20 in the first core 14 (excluding the non-sprayed region 20N) was 300 g/m$^2$, and the length L1 of the first core 14 in the lateral direction Y was 110 mm. In FIG. 6(c), the length represented by the reference numeral L2 (the length of the non-sprayed region 20N in the longitudinal direction X) was 200 mm, the length represented by the reference numeral L3 (the length of the non-sprayed region 20N in the width direction) was 10 mm, the length represented by the reference numeral L4 (the shortest distance between the pair of non-sprayed regions 20N) was 20 mm, the length represented by the reference numeral L5 (the largest distance in the lateral direction Y between the side edge of the first core 14 in the longitudinal direction X and the non-sprayed region 20N close to the side edge) was 35 mm, and the length represented by the reference numeral L6 (the largest distance between the pair of non-sprayed regions 20N) was 50 mm.

For each of the absorbent members in the disposable diapers of the example and comparative examples, by the following methods, a flexural rigidity $B_d$ of the laminate structure on the first core side (the laminate structure consisting of the first sheet, the first core, and the intermediate sheet) before liquid absorption in each of the longitudinal direction X, the lateral direction Y, and the oblique direction D, a flexural rigidity $B_w$ of the same laminate structure after liquid absorption in the same directions, and a thickness variation $T0_c$ before and after liquid absorption (= $T0_w$ - $T0_d$) were measured, and BR was calculated by the Formula (1) described above.

In addition, the absorbent assembly of each of the disposable diapers of the example and comparative examples was evaluated in terms of flexibility after liquid absorption by the following method.

Furthermore, the absorbent member of each of the disposable diapers of the example and comparative examples was evaluated in terms of liquid absorptivity by the following method.

The results are shown in the following Table 1.

<Measurement method of flexural rigidity>

It is generally known that the flexural rigidity of an object such as an absorbent member can be expressed as a value measured using KES (Kawabata Evaluation System) manufactured by Kato Tech Co., Ltd. (Reference: Sueo Kawabata, "Standardization and Analysis of Texture Evaluation", 2nd Edition, The Textile Machinery Society of Japan, Texture Measurement and Standardization Research Committee, published on July 10, 1980). Specifically, by using KES-FB2-AUTO-A (pure bending tester) and KES-FB2-AUTO-L (large bending tester) manufactured by Kato Tech Co., Ltd., it is possible to measure the flexural rigidity $B_d$ before liquid absorption and the flexural rigidity $B_w$ after liquid absorption for the laminate structure on the first core side. The measurement procedure is as follows.

(Measurement method of flexural rigidity $B_d$ before liquid absorption)

As a measurement device, KES-FB2-AUTO-A (pure bending tester) manufactured by Kato Tech Co., Ltd. is used. The sample prepared by the following <Sample preparation method> is mounted on the test table of the measurement device and gripped by chucks at an interval of 1 cm. The sample is subjected to pure bending at a constant velocity with a curvature in a range of curvature K = -2.5 to +2.5 $cm^{-1}$. One deformation cycle is performed at a deformation rate of 0.50 $cm^{-1}$/sec. The value of flexural rigidity $B_d$ before liquid absorption is calculated from the average of the slopes of the bending moments at a curvature between 0.5 and 1.5 and a curvature between -0.5 and -1.5. The flexural rigidity $B_d$ before liquid absorption is measured in each of the longitudinal direction X, the lateral direction Y, and the oblique direction D.

(Measurement method of flexural rigidity $B_w$ after liquid absorption)

As a measurement device, KES-FB2-AUTO-L (large bending tester) manufactured by Kato Tech Co., Ltd. is used. The sample prepared by the following <Sample preparation method> is immersed in physiological saline for 30 minutes, and then water is wiped off the sample by using an absorbent sheet such as Kim towel such that water does not leak from the sample, thereby preparing a sample after liquid absorption. The sample after liquid absorption is mounted on the test table of the measurement device and gripped by chucks at an interval of 4 cm. The sample after liquid absorption is bent at a constant velocity with a curvature in a range of curvature K = -0.4 to +0.4 $cm^{-1}$. One deformation cycle is performed at a deformation rate of 0.50 $cm^{-1}$/sec. The value of flexural rigidity $B_w$ after liquid absorption is calculated from the average of the slopes of the bending moments at a curvature between 0.1 and 0.3 and a curvature between -0.1 and -0.3. The flexural rigidity $B_w$ after liquid absorption is measured in each of the longitudinal direction X, the lateral direction Y, and the oblique direction D.

<Measurement method of thickness variation $T0_c$ before and after liquid absorption>

As a measurement device, a KES-G5 handy compression tester manufactured by Kato Tech Co., Ltd. is used. The sample is mounted on the test stand of the measurement device and compressed between chipboards having a circular surface with an area of 2 $cm^2$ such that a load of 4.9 mN/$cm^2$ (= 0.5 gf/$cm^2$) is applied to the sample, and in this state, the thickness of the sample is measured. The compression rate is 0.5 mm/sec, and the maximum compression load is 50 g/$cm^2$.

In a case where a thickness $T0_d$ of the laminate structure on the first core side before liquid absorption is measured under a load of 4.9 mN/$cm^2$, the sample prepared by the following <Sample preparation method> (dry sample) is used as it is as a sample.

In a case where a thickness $T0_w$ of the laminate structure on the first core side after liquid absorption is measured under a load of 4.9 mN/$cm^2$, the sample prepared by the following <Sample preparation method> (dry sample) is immersed in physiological saline for 30 minutes, water is then wiped off the sample by using an absorbent sheet such as Kim towel such that water does not leak from the sample, and the obtained sample (sample after liquid absorption) is used as a sample.

By subtracting the thickness $T0_c$ before liquid absorption from the thickness $T0_w$ after liquid absorption ($T0_w$ - $T0_d$), the thickness variation $T0_c$ before and after liquid absorption is calculated.

<Sample preparation method>

From the absorbent article, a measurement target (the laminate structure on the first core side: the laminate structure consisting of the first sheet, the first core, and the intermediate sheet) is collected. In a case where the measurement target is joined with another member by an adhesive, for example, by a method of exposing the joined portion to cold air from a cold spray, the adhesive force is removed, and then the measurement target is collected. This procedure is common to all measurement processes in the present description.

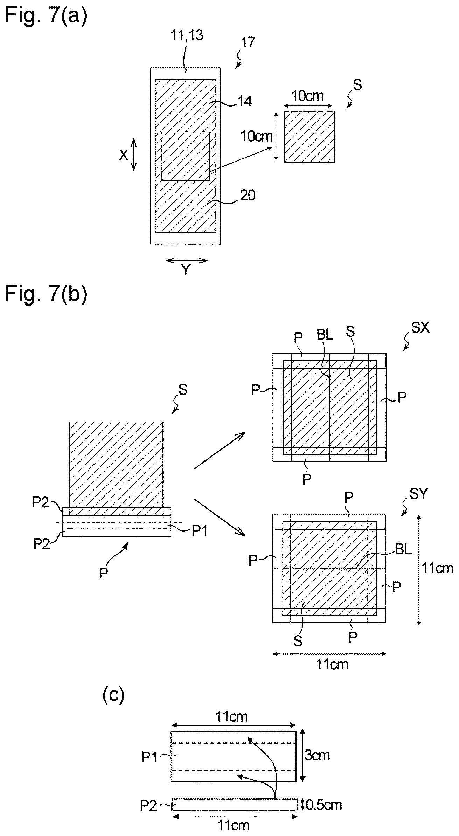

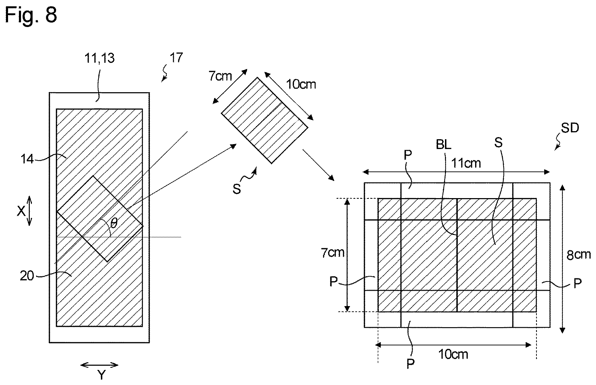

A sample is prepared from the measurement target according to the following procedure. FIGS. 7 and 8 show the procedure of preparing the sample. FIG. 7 shows the procedure of preparing samples SX and SY for measuring the flexural rigidity $B_d$ and $B_w$ in the longitudinal direction X or the lateral direction Y. FIG. 8 shows the procedure of preparing a sample SD for measuring the flexural rigidity $B_d$ and $B_w$ in the oblique direction D.

In order to prepare the sample SX for measuring the flexural rigidity in the longitudinal direction X and the sample SY for measuring the flexural rigidity in the lateral direction Y, with reference to FIG. 7, the laminate structure 17 on the first core side (the laminate structure consisting of the first sheet 11, the first core 14, and the intermediate sheet 13) as a measurement target is cut in the form of a square 10 cm long in the longitudinal direction X and 10 cm long in the lateral direction Y when seen in a plan view (see FIG. 7(a)) such that a slice S is obtained, and a protective sheet P that is separately prepared is attached to each of four sides of the slice S, thereby obtaining the samples SX and SY (see FIG. 7(b)). The protective sheet P consists of a substrate sheet P1 that is in the form of a rectangle (11 cm x 3 cm) when seen in a plan view and a pair of adhesive members P2 that is attached to lateral side edge portions of one surface of the substrate sheet P1 in the lengthwise direction (see FIG. 7(c)). Each adhesive member P2 consists of an adhesive such as a hot melt adhesive and is in the form of a rectangle (11 cm $\times$ 0.5 cm) when seen in a plan view. One of the pair of adhesive members P2 is used to be attached to the slice S, and the other adhesive member P2 extends outward from the slice S together with the substrate sheet P1 and is used to fix the samples SX and SY to the measurement device (test table) at the time of measuring the flexural rigidity. Each of the samples SX and SY is in the form of a square when seen in a plan view, and the length of one side thereof is 11 cm. The line represented by the reference numeral BL in FIG. 7 is a bending line along which the sample is bent at the time of measuring the bending rigidity.

In order to prepare the sample SD for measuring the flexural rigidity in the oblique direction D, with reference to FIG. 8, the laminate structure 17 on the first core side as a measurement target is cut in the form of a rectangle that is 7 cm long in the direction intersecting with the lateral direction Y at an angle $\theta$ (sharp angle) of 45 degrees, that is, in the oblique direction D, and 11 cm long in the direction orthogonal to the oblique direction D when seen in a plan view such that the slice S is obtained, and the protective sheet P is attached to each of four sides of the slice S in the same manner as described above, thereby obtaining the sample SD. The slice S may have a short side along the oblique direction D, and the length of the short side can be appropriately changed depending on the size of the laminate structure 17 on the first core side and the like. The length of the short side does not need to be 7 cm, and may be 3 cm, for example.

The material and basis weight of the substrate sheet P1 can be arbitrarily set within a range that does not affect the measurement of flexural rigidity. Examples of the material include nonwoven fabric, a resin film, and the like. The basis weight is about 5 to 20 g/m$^2$. Specific examples of the substrate sheet P1 include a spunbond nonwoven fabric having a basis weight of 17 g/m$^2$.

The basis weight and application pattern of the adhesive in the adhesive member P2 can be arbitrarily set within a range which does not affect the measurement of flexural rigidity and in which the protective sheet P is not detached after the samples SX, SY, and SD absorb a liquid (a range in which the absorbent polymer in the slice S does not leak). Specific examples of the adhesive member P2 include a substance obtained by applying a hot melt adhesive by a slot spray method such that the basis weight as a solid content reaches 6 g/m$^2$.

<Evaluation method of flexibility after liquid absorption>

An absorbent assembly (a laminate of the topsheet, the absorbent member, and the backsheet) was collected from the absorbent article (disposable diaper) as an evaluation target, and 160 g of physiological saline was injected for 1 minute into the central portion of the skin-facing surface (topsheet side) of the absorbent assembly. Thereafter, the absorbent assembly was left to stand for 30 minutes and then folded in half in the longitudinal direction (lengthwise direction) with the skin-facing surface facing inward. Ten specialized panelists were asked to freely touch the folded absorbent assembly with their fingers, mainly the folded part and the vicinity thereof, and asked to evaluate the softness of the absorbent assembly on the basis of 10 points. The average of the evaluation grades given by the ten panelists was adopted as the evaluation grade for the flexibility of the evaluation target after liquid absorption. The higher the evaluation grade, the better the flexibility in a liquid absorption state is decided to be, and the flexibility is evaluated as high.

<Evaluation method of liquid absorptivity>

The absorbent member was collected from the absorbent article (disposable diaper) as an evaluation target and placed on a slope having an inclination angle of 30° with the second core side of the absorbent member facing up. At this time, the longitudinal direction (lengthwise direction) of the absorbent member was made coincide with the inclination direction of the slope, and the front side (stomach side) of the absorbent member was placed on the lower side in the inclination direction. Physiological saline was repeatedly injected five times into the central portion of the upper surface of the absorbent member placed on the slope as described above, at an interval of 5 minutes in an amount of 40 g per injection (total injection amount: 200 g). Thereafter, the weight of the absorbent member (weight after liquid absorption) was measured, a liquid absorption amount (g) was calculated by subtracting the weight of the absorbent member before the injection of physiological saline from the weight after liquid absorption, and the liquid absorptivity was evaluated according to the following evaluation criteria.

(Evaluation criteria for liquid absorptivity)

A (excellent): The amount of liquid absorbed after 5 times of injection is 160 g or more.
B (slightly poor): The amount of liquid absorbed after 5 times of injection is 100 g or more and less than 160 g.
C (poor): The amount of liquid absorbed after 5 times of injection is less than 100 g.

[Table 1]

| | | Example | Comparative Example | |
|---|---|---|---|---|
| | | 1 | 1 | 2 |
| First core | Absorbent polymer occupancy (% by mass) | 100 | 100 | 100 |
| | Basis weight of absorbent polymer (g/m$^2$) | 110 | 100 | 300 |
| Laminate structure on first core side | Thickness $T0_d$ before liquid absorption (mm) | 1.3 | 1.2 | 1.8 |
| | Thickness $T0_w$ after liquid absorption (mm) | 8.1 | 5.9 | 15.3 |
| | Thickness variation before and after liquid absorption $T0_w$ - $T0_d$ (mm) | 6.8 | 4.7 | 13.5 |
| Flexural rigidity variation rate BR of laminate structure on first core side per thickness variation before and after liquid absorption | Longitudinal direction | 4.48 | 5.59 | 0.56 |
| | Lateral direction | 3.41 | 9.85 | 28.21 |
| | Oblique direction | 3.10 | 10.11 | 12.14 |
| Ratio between values of flexural rigidity $B_w$ of laminate structure on first core side in two directions after liquid absorption | $B_w$ in longitudinal direction/$B_w$ in lateral direction | 0.9 | 0.5 | 0.0 |
| | $B_w$ in longitudinal direction/$B_w$ in oblique direction | 1.0 | 0.6 | 0.1 |
| | $B_w$ in lateral direction/$B_w$ in oblique direction | 1.1 | 1.3 | 1.5 |
| Second core | Absorbent polymer occupancy (% by mass) | 61 | 61 | 61 |
| | Basis weight of absorbent polymer (g/m$^2$) | 230 | 230 | 230 |
| | Content of absorbent fiber (% by mass) | 39 | 39 | 39 |
| | Whether or not low-rigidity portion (through hole) exists | Exist | Exist | Exist |
| | Whether sheets are joined together in region overlapping low-rigidity portion when seen in a plan view | Joined | Joined | Joined |

(continued)

| | | Example | Comparative Example | |
|---|---|---|---|---|
| | | 1 | 1 | 2 |
| Evaluation | Flexibility after liquid absorption (based on 10 points) | 8.6 | 5.3 | 5.6 |
| | Liquid absorptivity | A | B | B |

Industrial Applicability

According to the present invention, there are provided an absorbent member that has excellent liquid absorption performance, is inhibited from becoming more rigid after liquid absorption, and exhibits excellent flexibility even after liquid absorption, and an absorbent article that includes the absorbent member.

## Claims

**1.** An absorbent member comprising:

a first sheet and a second sheet that face each other; and
an intermediate sheet that is interposed between the first sheet and the second sheet,
wherein
a first core that contains at least an absorbent polymer as an absorbent material is interposed between the first sheet and the intermediate sheet,
a second core that contains at least an absorbent fiber and an absorbent polymer as an absorbent material is interposed between the intermediate sheet and the second sheet,
a proportion of the absorbent polymer in a forming material of the first core is 80% by mass or more,
a ratio of a mass of the absorbent polymer contained in the first core to a total mass of the forming material of the first core is higher than a ratio of a mass of the absorbent polymer contained in the second core to a total mass of a forming material of the second core,
a basis weight of the absorbent polymer in the first core is smaller than a basis weight of the absorbent polymer in the second core, and
, wherein the flexural rigidity variation rate being calculated by the following Formula (1), BR in each of two directions randomly selected from three directions consisting of two directions that are orthogonal to each other and another direction that intersects with the two directions without being orthogonal to the two directions is 5.0 or less, wherein BR represents a flexural rigidity variation rate of a laminate structure consisting of the first sheet, the first core, and the intermediate sheet per thickness variation before and after liquid absorption,

$$BR = (B_w/B_d)/T0_c \qquad (1)$$

$B_w$: a flexural rigidity of the laminate structure after liquid absorption,
$B_d$: a flexural rigidity of the laminate structure before liquid absorption,
$T0_c$: a thickness variation of the laminate structure before and after liquid absorption calculated by the following Formula (2),

$$T0_c = T0_w - T0_d \qquad (2)$$

$T0_w$: a thickness of the laminate structure under a load of 4.9 mN/cm$^2$ after liquid absorption,
$T0_d$: a thickness of the laminate structure under a load of 4.9 mN/cm$^2$ before liquid absorption.

**2.** The absorbent member according to claim 1, wherein the ratio of the mass of the absorbent polymer contained in the first core to the total mass of the forming material of the first core/the ratio of the mass of the absorbent polymer contained in the second core to the total mass of the forming material of the second core is 1.1 or more and 5.0 or less.

**3.** The absorbent member according to any one of claims 1 to 2, wherein the ratio of the mass of the absorbent polymer contained in the second core to the total mass of the forming material of the second core is 30% by mass or more and90% by mass or less.

4. The absorbent member according to any one of claims 1 to 3, wherein on a premise that the basis weight of the absorbent polymer in the first core < the basis weight of the absorbent polymer in the second core, a ratio of the basis weight of the absorbent polymer in the second core/the basis weight of the absorbent polymer in the first core is 1.1 or more and 10.0 or less.

5. The absorbent member according to any one of claims 1 to 4, wherein the basis weight of the absorbent polymer in the first core is 60 g/m$^2$ or more and 700 g/m$^2$ or less.

6. The absorbent member according to any one of claims 1 to 5, wherein the basis weight of the absorbent polymer in the second core is 65 g/m$^2$ or more. and 800 g/m$^2$ or less.

7. The absorbent member according to any one of claims 1 to 6, wherein the BR in each of two directions randomly selected from the three directions is 0.1 or more and 4.8 or less.

8. The absorbent member according to any one of claims 1 to 7, wherein the BR in each of the three directions is 5.0 or less.

9. The absorbent member according to any one of claims 1 to 8, wherein a flexural rigidity $B_w$ of the laminate structure in one direction after liquid absorption is 0.3 mN·cm$^2$/cm or more and 200 mN·cm$^2$/cm or less.

10. The absorbent member according to any one of claims 1 to 9, wherein the absorbent member is used as an absorbent member of an absorbent article to be placed in a crotch portion of a wearer, and
in a region of the first core that is to be placed in the crotch portion when the absorbent article is worn, the absorbent polymer is evenly distributed.

11. The absorbent member according to any one of claims 1 to 10, wherein the absorbent member has a shape long in one direction when seen in a plan view, and
based on a center of the absorbent member in a longitudinal direction as a boundary, one side of the absorbent member in the longitudinal direction is richer in the absorbent material than the other side of the absorbent member in the longitudinal direction.

12. The absorbent member according to any one of claims 1 to 11, wherein the absorbent member is used as an absorbent member of an absorbent article to be placed in a crotch portion of a wearer, and
the absorbent member is placed so that a side of the absorbent member in a longitudinal direction in which the absorbent material is concentrated is located close to a front portion of the absorbent article.

13. The absorbent member according to any one of claims 1 to 12, wherein adhesives are applied to surfaces of the first sheet and the intermediate sheet, the surfaces facing the first core,

one of the adhesives is applied to a side of a liquid-receiving surface of the first core that firstly comes into contact with a liquid to be absorbed so that a coated region of the adhesive includes both a coated portion and a non-coated portion, and
one of the adhesives is applied to an opposite side of the side of the liquid-receiving surface so that a non-coated portion substantially does not exist in a coated region of the adhesive.

14. The absorbent member according to any one of claims 1 to 13, wherein the second core has a low-rigidity portion in which the forming material of the second core does not exist or the forming material of the second core having a basis weight lower than a basis weight of the forming material of the second core in a peripheral portion exists.

15. An absorbent article comprising:
the absorbent member according to any one of claims 1 to 14.

## Patentansprüche

1. Absorbierendes Element, welches umfasst:

eine erste Lage und eine zweite Lage, die einander zugewandt sind; und
eine Zwischenlage, die zwischen der ersten Lage und der zweiten Lage angeordnet ist,
wobei
ein erster Kern, der zumindest ein absorbierendes Polymer als absorbierendes Material enthält, zwischen der ersten Lage und der Zwischenlage angeordnet ist,
ein zweiter Kern, der zumindest eine absorbierende Faser und ein absorbierendes Polymer als absorbierendes Material enthält, zwischen der Zwischenlage und der zweiten Lage angeordnet ist,
ein Anteil des absorbierenden Polymers in einem Material, aus dem der ersten Kern gebildet ist, 80 Gew.-% oder mehr beträgt,
ein Verhältnis einer Masse des in dem ersten Kern enthaltenen absorbierenden Polymers zu der Gesamtmasse des Materials, aus dem der erste Kern gebildet ist, größer ist als ein Verhältnis der Masse des in dem zweiten Kern enthaltenen absorbierenden Polymers zu der Gesamtmasse eines Materials, aus dem der zweite Kern gebildet ist,
ein Grundgewicht des absorbierenden Polymers in dem ersten Kern geringer ist als ein Grundgewicht des absorbierenden Polymers in dem zweiten Kern, und
wobei die Biegesteifigkeitsänderungsrate durch die folgende Formel (1) berechnet wird, BR in jeder von zwei zufällig aus drei Richtungen ausgewählten Richtungen, die aus zwei zueinander orthogonalen Richtungen und einer weiteren Richtung bestehen, die die beiden Richtungen schneidet, ohne zu den beiden Richtungen orthogonal zu sein, 5,0 oder weniger beträgt, wobei BR ein Verhältnis der Änderung der Biegesteifigkeit einer Schichtstruktur darstellt, die aus der ersten Lage, dem ersten Kern und der Zwischenlage besteht, durch Änderung der Dicke vor und nach der Flüssigkeitsaufnahme,

$$BR = (B_w / B_d) / T0_c \quad (1)$$

$B_w$ : eine Biegesteifigkeit der Schichtstruktur nach Flüssigkeitsaufnahme,
$B_d$ : Biegesteifigkeit der Schichtstruktur vor der Flüssigkeitsaufnahme,
$T0_c$ : Veränderung der Dicke der Schichtstruktur vor und nach der Flüssigkeitsaufnahme, berechnet nach der folgenden Formel (2)

$$T0_c = T0_w - T0_d \quad (2)$$

TOw: Dicke der Schichtstruktur unter einer Belastung von 4,9 mN/cm$^2$ nach Flüssigkeitsaufnahme,
$T0_d$: Dicke der Schichtstruktur unter einer Belastung von 4,9 mN/cm$^2$ vor der Flüssigkeitsaufnahme.

**2.** Absorbierendes Element nach Anspruch 1, wobei das Verhältnis der Masse des in dem ersten Kern enthaltenen absorbierenden Polymers zu der Gesamtmasse des Materials, aus dem der erste Kern gebildet ist/das Verhältnis der Masse des in dem zweiten Kern enthaltenen absorbierenden Polymers zu der Gesamtmasse des Materials, aus dem der zweite Kern gebildet ist, 1,1 oder mehr und 5,0 oder weniger beträgt.

**3.** Absorbierendes Element nach einem der Ansprüche 1 bis 2, wobei das Verhältnis der Masse des in dem zweiten Kern enthaltenen absorbierenden Polymers zu der Gesamtmasse des Materials, aus dem der zweite Kern gebildet ist, 30 Massenprozent oder mehr und 90 Massenprozent oder weniger beträgt.

**4.** Absorbierendes Element nach einem der Ansprüche 1 bis 3, wobei unter der Annahme, dass das Grundgewicht des absorbierenden Polymers in dem ersten Kern < zu dem Grundgewicht des absorbierenden Polymers in dem zweiten Kern beträgt, ein Verhältnis des Grundgewichts des absorbierenden Polymers in dem zweiten Kern zu dem Grundgewicht des absorbierenden Polymers in dem ersten Kern 1,1 oder mehr und 10,0 oder weniger beträgt.

**5.** Absorbierendes Element nach einem der Ansprüche 1 bis 4, wobei das Grundgewicht des absorbierenden Polymers in dem ersten Kern 60 g/m$^2$ oder mehr und 700 g/m$^2$ oder weniger beträgt.

**6.** Absorbierendes Element nach einem der Ansprüche 1 bis 5, wobei das Grundgewicht des absorbierenden Polymers in dem zweiten Kern 65 g/m$^2$ oder mehr und 800 g/m$^2$ oder weniger beträgt.

**7.** Absorbierendes Element nach einem der Ansprüche 1 bis 6, wobei der BR in jeder der beiden zufällig aus den drei Richtungen ausgewählten Richtungen 0,1 oder mehr und 4,8 oder weniger beträgt.

**8.** Absorbierendes Element nach einem der Ansprüche 1 bis 7, wobei der BR in jeder der drei ausgewählten Richtungen 5,0 oder weniger beträgt.

**9.** Absorbierendes Element nach einem der Ansprüche 1 bis 8, wobei die Biegesteifigkeit $B_w$ der Schichtstruktur in einer Richtung nach der Flüssigkeitsaufnahme 0,3 mN·cm$^2$/cm oder mehr und 200 mN·cm$^2$/cm oder weniger beträgt.

**10.** Absorbierendes Element nach einem der Ansprüche 1 bis 9, wobei das absorbierende Element als absorbierendes Element eines absorbierenden Artikels verwendet wird, der in einem Schrittbereich eines Trägers angeordnet werden soll, und
in einem Bereich des ersten Kerns, der beim Tragen des absorbierenden Artikels in dem Schrittbereich angeordnet werden soll, das absorbierende Polymer gleichmäßig verteilt ist.

**11.** Absorbierendes Element nach einem der Ansprüche 1 bis 10, wobei das absorbierende Element in einer Draufsicht eine längliche Form aufweist, und
auf Grundlage einer Mitte des absorbierenden Elements in einer Längsrichtung als Grenze eine Seite des absorbierenden Elements in der Längsrichtung mehr absorbierendes Material enthält als die andere Seite des absorbierenden Elements in der Längsrichtung.

**12.** Absorbierendes Element nach einem der Ansprüche 1 bis 11, wobei das absorbierende Element als absorbierendes Element eines absorbierenden Artikels verwendet wird, der in dem Schrittbereich eines Trägers angeordnet werden soll, und
das absorbierende Element derart platziert wird, dass eine Seite des absorbierenden Elements in einer Längsrichtung, in der das absorbierende Material konzentriert ist, in der Nähe eines vorderen Abschnitts des absorbierenden Artikels liegt.

**13.** Absorbierendes Element nach einem der Ansprüche 1 bis 12, wobei Klebstoffe auf Oberflächen der ersten Lage und der Zwischenlage aufgebracht sind, wobei die Oberflächen dem ersten Kern zugewandt sind,

einer der Klebstoffe auf eine Seite einer Flüssigkeitsaufnahmefläche des ersten Kerns aufgebracht wird, die zuerst mit einer zu absorbierenden Flüssigkeit in Kontakt kommt, so dass ein mit dem Klebstoff beschichteter Bereich sowohl einen beschichteten als auch einen unbeschichteten Teil aufweist, und
einer der Klebstoffe auf eine der Seite der Flüssigkeitsaufnahmefläche gegenüberliegende Seite aufgebracht wird, so dass in einem mit dem Klebstoff beschichteten Bereich im Wesentlichen kein unbeschichteter Teil vorhanden ist.

**14.** Absorbierendes Element nach einem der Ansprüche 1 bis 13, wobei der zweite Kern einen Bereich mit geringer Steifigkeit aufweist, in dem das Material, aus dem der zweite Kern gebildet ist, nicht vorhanden ist oder das Material, aus dem der zweite Kern gebildet ist, das ein geringeres Grundgewicht als das Grundgewicht des Materials aufweist, aus dem der zweite Kern gebildet ist, in einem peripheren Bereich vorhanden ist.

**15.** Absorbierender Artikel, welcher umfasst:
das absorbierende Element nach einem der Ansprüche 1 bis 14.

## Revendications

**1.** Elément absorbant comprenant :

une première feuille et une seconde feuille se faisant face ; et
une feuille intermédiaire qui est intercalée entre la première feuille et la seconde feuille,
dans lequel
un premier noyau qui contient au moins un polymère absorbant comme matériau absorbant est intercalée entre la première feuille et la feuille intermédiaire,
un second noyau qui contient au moins une fibre absorbante et un polymère absorbant en tant que matériau absorbant est intercalé entre la feuille intermédiaire et la seconde feuille,
une proportion du polymère absorbant dans un matériau de formation du premier noyau est de 80 % en poids ou plus,
un rapport d'une masse du polymère absorbant contenu dans le premier noyau sur une masse totale du matériau de formation du premier noyau est supérieur à un rapport d'une masse du polymère absorbant contenu dans le second noyau sur une masse totale d'un matériau de formation du second noyau,

un poids de base du polymère absorbant dans le premier noyau est inférieur à un poids de base du polymère absorbant dans le second noyau, et

dans lequel le taux de variation de rigidité à la flexion étant calculé par la formule suivante (1), BR dans chacune de deux directions sélectionnées de manière aléatoire parmi trois directions constituées de deux directions qui sont orthogonales entre elles et d'une autre direction qui croise les deux directions sans être orthogonale aux deux directions est de 5,0 ou moins, dans lequel BR représente un taux de variation de rigidité à la flexion d'une structure stratifiée composée de la première feuille, du premier noyau et de la feuille intermédiaire par variation d'épaisseur avant et après absorption de liquide,

$$BR = (B_w/B_d) / T0_c \ (1)$$

$B_w$ : une rigidité à la flexion de la structure stratifiée après absorption de liquide,

$B_d$: une rigidité à la flexion de la structure stratifiée avant absorption de liquide,

$T0_c$ : variation d'épaisseur de la structure stratifiée avant et après l'absorption de liquide calculée par la formule (2) suivante,

$$T0_c = T0_w - T0_d \ (2)$$

T0w : une épaisseur de la structure stratifiée sous une charge de 4,9 mN/cm$^2$ après absorption du liquide,

$T0_d$ : une épaisseur de la structure stratifiée sous une charge de 4,9 mN/cm$^2$ avant absorption du liquide.

**2.** Élément absorbant selon la revendication 1, dans lequel le rapport de la masse du polymère absorbant contenu dans le premier noyau sur la masse totale du matériau de formation du premier noyau /le rapport de la masse du polymère absorbant contenu dans le second noyau sur la masse totale du matériau de formation du second noyau est de 1,1 ou plus et de 5,0 ou moins.

**3.** Élément absorbant selon l'une quelconque des revendications 1 à 2, dans lequel le rapport de la masse du polymère absorbant contenu dans le second noyau sur la masse totale du matériau de formation du second noyau est de 30 % en masse ou plus et de 90 % en masse ou moins.

**4.** Élément absorbant selon l'une quelconque des revendications 1 à 3, dans lequel, en supposant que le poids de base du polymère absorbant dans le premier noyau < au poids de base du polymère absorbant dans le second noyau, un rapport du poids de base du polymère absorbant dans le second noyau/le poids de base du polymère absorbant dans le premier noyau est de 1,1 ou plus et de 10,0 ou moins.

**5.** Élément absorbant selon l'une quelconque des revendications 1 à 4, dans lequel le poids de base du polymère absorbant dans le premier noyau est de 60 g/m$^2$ ou plus et de 700 g/m$^2$ ou moins.

**6.** Élément absorbant selon l'une quelconque des revendications 1 à 5, dans lequel le poids de base du polymère absorbant dans le second noyau est de 65 g/m$^2$ ou plus et de 800 g/m$^2$ ou moins.

**7.** Élément absorbant selon l'une quelconque des revendications 1 à 6, dans lequel le BR dans chacune des deux directions sélectionnées de manière aléatoire parmi les trois directions est de 0,1 ou plus et de 4,8 ou moins.

**8.** Élément absorbant selon l'une quelconque des revendications 1 à 7, dans lequel le BR dans chacune des trois directions sélectionnées est de 5,0 ou moins.

**9.** Élément absorbant selon l'une quelconque des revendications 1 à 8, dans lequel la rigidité à la flexion $B_w$ de la structure stratifiée dans une direction après absorption de liquide est de 0,3 mN·cm$^2$/cm ou plus et de 200 mN·cm$^2$/cm ou moins.

**10.** Élément absorbant selon l'une quelconque des revendications 1 à 9, dans lequel l'élément absorbant est utilisé comme élément absorbant d'un article absorbant destiné à être placé dans une partie de l'entrejambe d'un porteur, et dans une région du premier noyau qui doit être placée dans la partie de l'entrejambe lorsque l'article absorbant est porté, le polymère absorbant est réparti uniformément.

**11.** Élément absorbant selon l'une quelconque des revendications 1 à 10, dans lequel l'élément absorbant présente une forme longue dans une direction vu dans une vue en plan, et
sur la base d'un centre de l'élément absorbant dans une direction longitudinale en tant que limite, un côté de l'élément absorbant dans la direction longitudinale est plus riche en matériau absorbant que l'autre côté de l'élément absorbant dans la direction longitudinale.

**12.** Elément absorbant selon l'une quelconque des revendications 1 à 11, dans lequel l'élément absorbant est utilisé comme un élément absorbant d'un article absorbant devant être placé dans la partie de l'entrejambe d'un porteur, et l'élément absorbant est placé de telle sorte qu'un côté de l'élément absorbant dans une direction longitudinale dans laquelle le matériau absorbant est concentré est situé à proximité d'une partie avant de l'article absorbant.

**13.** Élément absorbant selon l'une quelconque des revendications 1 à 12, dans lequel des adhésifs sont appliqués sur des surfaces de la première feuille et de la feuille intermédiaire, les surfaces faisant face au premier noyau,

un des adhésifs est appliqué sur un côté d'une surface de réception de liquide du premier noyau qui vient tout d'abord en contact avec un liquide à absorber de telle sorte qu'une région revêtue de l'adhésif comporte à la fois une partie revêtue et une partie non revêtue, et
un des adhésifs est appliqué sur un côté opposé du côté de la surface de réception de liquide de telle sorte qu'une partie non revêtue n'existe sensiblement pas dans une région revêtue de l'adhésif.

**14.** Élément absorbant selon l'une quelconque des revendications 1 à 13, dans lequel le second noyau présente une partie à faible rigidité dans laquelle le matériau de formation du second noyau n'existe pas ou le matériau de formation du second noyau présentant un poids de base inférieur à un poids de base du matériau de formation du second noyau dans une partie périphérique existe.

**15.** Article absorbant comprenant :
l'élément absorbant selon l'une quelconque des revendications 1 à 14.

Fig. 1

1
5
31
3
2
5
7
8
62
15
13(14)
8
4
6
61
10
15B
15N
I
I
32
32
60
60
62
X
15A
9
31
CLy
Y
C
B
A

Fig. 2

61
61
6
6
20
3
15N
15
15N
12
60
60
32
32
13
4
14
11
12E
20
10
5
2
5
Y

Fig. 3

L0
15A
15B
15
L
CLx
13(14)
10
G
CLy
W
Y
12
15N
X

Fig. 4

10
20
15N
20
15N
12
20
25
24
15
13
14
23
22
21
20
12E
21
12E
20
11
Y

Fig. 5(a)

10
15N
20
20
12
25
15
24
13
23
14
22
11
26
(22, 23)
26
(22, 23)
20
26
(22, 23)
20
Y

Fig. 5(b)

15N
10
12
24
20
20
25
15
24
13
23
14
22
16
11
26
(22, 23)
20
26
(22, 23)
20
Y

Fig. 6(a)
17A
L1
14
X
20
11,13
Fig. 6(b)
17B
L1
L10
20
L10
20
20
14
11,13
Y
Fig. 6(c)
17C
L1
20N
L3
L5
L2
L4
20N
L6
14
11,13

Fig. 7(a)

11,13
17
14
10cm
S
10cm
X
20
Y

Fig. 7(b)

P
BL
S
SX
S
P
P
P
P2
P1
P2
P
P
SY
P
BL
11cm
P
S
P
11cm

(c)

11cm
P1
3cm
P2
0.5cm
11cm

Fig. 8

11,13
17
14
X
θ
20
Y
7cm
10cm
S
SD
BL
S
P
11cm
7cm
8cm
P
P
10cm
P

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018050987 A
- JP 2010529879 A
- WO 2010076857 A1

### Non-patent literature cited in the description

- **SUEO KAWABATA**. Standardization and Analysis of Texture Evaluation. The Textile Machinery Society of Japan, Texture Measurement and Standardization Research Committee, 10 July 1980